# EUROPEAN PATENT APPLICATION

(11) **EP 4 803 015 A1**
(43) Date of publication of application: **09.09.2026**
(21) Application number: 24885719.5
(22) Date of filing: 29.10.2024
(51) Int. Cl.: A61B 8/14, A61B 6/00, A61B 6/50, G06T 7/00, G06V 10/25, G06V 10/30

(54) **IMAGE PROCESSING SYSTEM, IMAGE PROCESSING METHOD, IMAGE PROCESSING PROGRAM, AND IMAGE PROCESSING DEVICE**

(30) Priority: 31.10.2023 JP 2023186484
(71) Applicant: CHUGAI SEIYAKU KABUSHIKI KAISHA, Tokyo 115-8543 (JP)
(72) Inventor: YANG Liu, Tokyo 103-8324 (JP); MURAKAMI Yoichi, Tokyo 103-8324 (JP); ITO Takahiro, Tokyo 103-8324 (JP); NAGAO Azusa, Tokyo 167-0035 (JP); INAGAKI Yusuke, Kashihara-shi, Nara 634-8521 (JP); TAKEDANI Hideyuki, Tokyo 108-8639 (JP)
(74) Representative: Vossius & Partner Patentanwälte Rechtsanwälte mbB
(86) International application number: PCT/JP2024/038561
(87) International publication number: WO 2025/094952

(57) **Abstract**

The purpose is to determine a more accurate region of interest. An image processing system 1 comprises a landmark detection unit 43 that detects, in a joint image which is an in vivo image of a human joint, two or more joint landmarks, each of which is a landmark for the joint, and a ROI1 determination unit 44 that determines, in the joint image, a region of interest for the joint based on the two or more joint landmarks detected. The landmark detection unit 43 may perform detection using a landmark detection model which, upon input of the joint image, detects two or more joint landmarks in the joint image. The landmark detection model may be a model trained based on training data comprising a pair of a joint image and an image in which two or more joint landmarks in the joint image are annotated.

## Description

### [Technical Field]

An aspect of the present disclosure relates to an image processing system, an image processing method, an image processing program and an image processing device for processing an image.

### [Background Art]

Patent Literature 1 described below discloses an information processing device that defines a square as a region of interest for each joint of a human body, the square having a predetermined size and centering on the joint.

### [Citation List]

### [Patent Literature]

[Patent Literature 1] Japanese Patent Laid-Open No. 2022-080113

### [Summary of Invention]

### [Technical Problem]

The information processing device, which defines a square having a predetermined size and centering on a joint, as a region of interest, may fail to define a region of interest in which the joint is accurately captured. Thus, determination of a more accurate region of interest is desired.

### [Solution to Problem]

An image processing system according to an aspect of the present disclosure comprises a landmark detection unit that detects, in a joint image which is an in vivo image of a human joint, two or more joint landmarks, each of which is a landmark for the joint, and a determination unit that determines, in the joint image, a region of interest for the joint based on the two or more joint landmarks detected. In this aspect, a region of interest is determined based on the two or more joint landmarks detected. In this way, a more accurate region of interest can be determined.

The landmark detection unit may perform detection using a landmark detection model which, upon input of the joint image, detects the two or more joint landmarks in the joint image. In this aspect, two or more joint landmarks can be more reliably and accurately detected by using a landmark detection model.

The landmark detection model may be a model trained based on training data comprising a pair of the joint image and an image in which the two or more joint landmarks in the joint image are annotated. In this aspect, the landmark detection model trained based on the training data is capable of more reliably and accurately detecting two or more joint landmarks.

The landmark detection unit may also detect types of the joint landmarks, and the determination unit may perform determination based further on the detected types of the joint landmarks. In this aspect, a region of interest is determined based further on types of the joint landmarks. In this way, a more accurate region of interest can be determined.

One of the two or more joint landmarks detected may be a bone, with another being a different bone, cartilage, fat tissue, a joint capsule, or muscle. In this aspect, a more accurate region of interest can be determined based on a more specific joint landmark.

The joint may be a joint of a hemophilia patient. In this aspect, a region of interest in a joint image of a hemophilia patient can be determined.

The joint image may be an ultrasound image or an X-ray image. In general, echography or X-ray radiography can be conveniently conducted on an outpatient basis, and therefore, in this aspect, a region of interest can be conveniently determined on an outpatient basis, for example.

The joint image may be an image having enhanced image quality. In this aspect, two or more joint landmarks can be more accurately detected based on a joint image having enhanced image quality. In this way, a more accurate region of interest can be determined.

The joint landmark may be a tibia, a talus, a fibula, a femur, a patella, a humerus, a radius, an ulna, cartilage, fat tissue, a joint capsule, or muscle. In this aspect, a more accurate region of interest can be determined based on a more specific joint landmark.

The determination unit may determine the region of interest based on a distance from a center of the joint landmark to a boundary line of the region of interest. In this aspect, a region of interest can be more reliably determined.

The image processing system may further comprise a state detection unit that detects a joint state which is a state of the joint, based on a local joint image which is an image of an inside of the determined region of interest in the joint image. In this aspect, a more accurate joint state can be detected based on a local joint image. In this way, a user can know a more accurate joint state.

The state detection unit may perform detection using a state detection model which, upon input of the local joint image, detects the joint state of the joint shown by the local joint image. In this aspect, a joint state can be more reliably and accurately detected by using a state detection model.

The state detection model may be a model trained based on training data comprising a pair of the local joint image and the local joint image with which a normality label is associated or the local joint image in which an abnormality label is associated with an abnormal area of the joint shown by the local joint image. In this aspect, the state detection model trained based on the training data is capable of more reliably and accurately detecting a joint state.

The image processing system may further comprise a visualization unit that visualizes a portion of the local joint image, which is influenced in detection of the joint state by the state detection model, in a display mode corresponding to a degree of the influence. In this aspect, a user can know a joint state in more detail in the display mode.

The joint state may indicate normality, abnormality, bleeding, synovitis or arthrosis. In this aspect, a more specific joint state can be output. In this way, a user can know a more specific joint state.

When indicating abnormality, bleeding, synovitis or arthrosis, the joint state may further indicate a relevant area in the local joint image. In this aspect, abnormality, bleeding, synovitis or arthrosis, as well as a relevant area in the local joint image can be output. In this way, a user can know a more specific joint state.

The joint may be a foot joint, the joint landmark may be a tibia, a fibula, a talus or fat tissue, and the joint state may indicate normality, abnormality, bleeding, synovitis or arthrosis. In this aspect, a more accurate region of interest can be determined based on two or more specific joint landmarks in a foot joint image. A specific joint state can be output based on the determined region of interest. In this way, a user can know a more accurate and specific joint state of a foot joint.

The joint may be a knee joint, the joint landmark may be a femur, a tibia, a fibula, a patella or fat tissue, and the joint state may indicate normality, abnormality, bleeding, synovitis or arthrosis. In this aspect, a more accurate region of interest can be determined based on two or more specific joint landmarks in a knee joint image. A specific joint state can be output based on the determined region of interest. In this way, a user can know a more accurate and specific joint state of a knee joint.

The joint may be an elbow joint, the joint landmark may be a humerus, a radius, an ulna or fat tissue, and the joint state may indicate normality, abnormality, bleeding, synovitis or arthrosis. In this aspect, a more accurate region of interest can be determined based on two or more specific joint landmarks in an elbow joint image. A specific joint state can be output based on the determined region of interest. In this way, a user can know a more accurate and specific joint state of an elbow joint.

Diagnosis of the joint state may be supported based on the joint image. In this aspect, diagnosis of a joint state can be supported based on a joint image.

An image processing program according to an aspect of the present disclosure causes a computer to function as a landmark detection unit that detects, in a joint image which is an in vivo image of a human joint, two or more joint landmarks, each of which is a landmark for the joint, and a determination unit that determines, in the joint image, a region of interest for the joint based on the two or more joint landmarks detected. In this aspect, a region of interest is determined based on the two or more joint landmarks detected. In this way, a more accurate region of interest can be determined.

The landmark detection unit may perform detection using a landmark detection model which, upon input of the joint image, detects the two or more joint landmarks in the joint image. In this aspect, two or more joint landmarks can be more reliably and accurately detected by using a landmark detection model.

The landmark detection model may be a model trained based on training data comprising a pair of the joint image and an image in which the two or more joint landmarks in the joint image are annotated. In this aspect, the landmark detection model trained based on the training data is capable of more reliably and accurately detecting two or more joint landmarks.

The landmark detection unit may also detect types of the joint landmarks, and the determination unit may perform determination based further on the detected types of the joint landmarks. In this aspect, a region of interest is determined based further on types of the joint landmarks. In this way, a more accurate region of interest can be determined.

One of the two or more joint landmarks detected may be a bone, with another being a different bone, cartilage, fat tissue, a joint capsule or muscle. In this aspect, a more accurate region of interest can be determined based on a more specific joint landmark.

The joint may be a joint of a hemophilia patient. In this aspect, a region of interest in a joint image of a hemophilia patient can be determined.

The joint image may be an ultrasound image or an X-ray image. In general, echography or X-ray radiography can be conveniently conducted on an outpatient basis, and therefore, in this aspect, a region of interest can be conveniently determined on an outpatient basis, for example.

The joint image may be an image having enhanced image quality. In this aspect, two or more joint landmarks can be more accurately detected based on a joint image having enhanced image quality. In this way, a more accurate region of interest can be determined.

The joint landmark may be a tibia, a talus, a fibula, a femur, a patella, a humerus, a radius, an ulna, cartilage, fat tissue, a joint capsule, or muscle. In this aspect, a more accurate region of interest can be determined based on a more specific joint landmark.

The determination unit may determine the region of interest based on a distance from a center of the joint landmark to a boundary line of the region of interest. In this aspect, a region of interest can be more reliably determined.

The computer may be caused to further function as a state detection unit that detects a joint state which is a state of the joint, based on a local joint image which is an image of an inside of the determined region of interest in the joint image. In this aspect, a more accurate joint state can be detected based on a local joint image. In this way, a user can know a more accurate joint state.

The state detection unit may perform detection using a state detection model which, upon input of the local joint image, detects the joint state of the joint shown by the local joint image. In this aspect, a joint state can be more reliably and accurately detected by using a state detection model.

The state detection model may be a model trained based on training data comprising a pair of the local joint image and the local joint image in which a normality label is associated with the joint shown by the local joint image or the local joint image in which an abnormality label is associated with an abnormal area of the joint shown by the local joint image. In this aspect, the state detection model trained based on the training data is capable of more reliably and accurately detecting a joint state.

The computer may be caused to function further as a visualization unit that visualizes a portion of the local joint image, which is influenced in detection of the joint state by the state detection model, in a display mode corresponding to a degree of the influence. In this aspect, a user can know a joint state in more detail in the display mode.

The joint state may indicate normality, abnormality, bleeding, synovitis or arthrosis. In this aspect, a more specific joint state can be output. In this way, a user can know a more specific joint state.

When indicating abnormality, bleeding, synovitis or arthrosis, the joint state may further indicate a relevant area in the local joint image. In this aspect, abnormality, bleeding, synovitis or arthrosis, as well as a relevant area in the local joint image can be output. In this way, a user can know a more specific joint state.

The joint may be a foot joint, the joint landmark may be a tibia, a fibula, a talus or fat tissue, and the joint state may indicate normality, abnormality, bleeding, synovitis or arthrosis. In this aspect, a more accurate region of interest can be determined based on two or more specific joint landmarks in a foot joint image. A specific joint state can be output based on the determined region of interest. In this way, a user can know a more accurate and specific joint state of a foot joint.

The joint may be a knee joint, the joint landmark may be a femur, a tibia, a fibula, a patella or fat tissue, and the joint state may indicate normality, abnormality, bleeding, synovitis or arthrosis. In this aspect, a more accurate region of interest can be determined based on two or more specific joint landmarks in a knee joint image. A specific joint state can be output based on the determined region of interest. In this way, a user can know a more accurate and specific joint state of a knee joint.

The joint may be an elbow joint, the joint landmark may be a humerus, a radius, an ulna or fat tissue, and the joint state may indicate normality, abnormality, bleeding, synovitis or arthrosis. In this aspect, a more accurate region of interest can be determined based on two or more specific joint landmarks in an elbow joint image. A specific joint state can be output based on the determined region of interest. In this way, a user can know a more accurate and specific joint state of an elbow joint.

Diagnosis of the joint state may be supported based on the joint image. In this aspect, diagnosis of a joint state can be supported based on a joint image.

An image processing method according to an aspect of the present disclosure is executed by a computer, and comprises a landmark detection step of detecting, in a joint image which is an in vivo image of a human joint, two or more joint landmarks, each of which is a landmark for the joint, and a determination step of determining, in the joint image, a region of interest for the joint based on the two or more joint landmarks detected. In this aspect, a region of interest is determined based on the two or more joint landmarks detected. In this way, a more accurate region of interest can be determined.

An image processing device according to an aspect of the present disclosure comprises a landmark detection unit that detects, in a joint image which is an in vivo image of a human joint, two or more joint landmarks, each of which is a landmark for the joint, and a determination unit that determines, in the joint image, a region of interest for the joint based on the two or more joint landmarks detected. In this aspect, a region of interest is determined based on the two or more joint landmarks detected. In this way, a more accurate region of interest can be determined.

The image processing device may further comprise a state detection unit that detects a joint state which is a state of the joint, based on a local joint image which is an image of an inside of the determined region of interest in the joint image. In this aspect, a more accurate joint state can be detected based on a local joint image. In this way, a user can know a more accurate joint state.

An image processing system, an image processing method, an image processing program and an image processing device according to an aspect of the present disclosure can be described as follows.
[1] An image processing system comprising:
   a landmark detection unit that detects, in a joint image which is an in vivo image of a human joint, two or more joint landmarks, each of which is a landmark for the joint; and
   a determination unit that determines, in the joint image, a region of interest for the joint based on the two or more joint landmarks detected.
[2] The image processing system according to [1], wherein the landmark detection unit performs detection using a landmark detection model which, upon input of the joint image, detects the two or more joint landmarks in the joint image.
[3] The image processing system according to [2], wherein the landmark detection model is a model trained based on training data comprising a pair of the joint image and an image in which the two or more joint landmarks in the joint image are annotated.
[4] The image processing system according to any one of [1] to [3], wherein the landmark detection unit also detects types of the joint landmarks, and
   the detection unit performs determination based further on the detected types of the joint landmarks.
[5] The image processing system according to any one of [1] to [4], wherein one of the two or more joint landmarks detected is a bone, and another is a different bone, cartilage, fat tissue, a joint capsule or muscle.
[6] The image processing system according to any one of [1] to [5], wherein the joint is a joint of a hemophilia patient.
[7] The image processing system according to any one of [1] to [6], wherein the joint image is an ultrasound image or an X-ray image.
[8] The image processing system according to any one of [1] to [7], wherein the joint image is an image having enhanced image quality.
[9] The image processing system according to any one of [1] to [8], wherein the joint landmark is a tibia, a talus, a fibula, a femur, a patella, a humerus, a radius, an ulna, cartilage, fat tissue, a joint capsule, or muscle.
[10] The image processing system according to any one of [1] to [9], wherein the determination unit determines the region of interest based on a distance from a center of the joint landmark to a boundary line of the region of interest.
[11] The image processing system according to any one of [1] to [10], further comprising a state detection unit that detects a joint state which is a state of the joint, based on a local joint image which is an image of an inside of the determined region of interest in the joint image.
[12] The image processing system according to [11], wherein the state detection unit performs detection using a state detection model which, upon input of the local joint image, detects the joint state of the joint shown by the local joint image.
[13] The image processing system according to [12], wherein the state detection model is a model trained based on training data comprising a pair of the local joint image and the local joint image with which a normality label is associated or the local joint image in which an abnormality label is associated with an abnormal area of the joint shown by the local joint image.
[14] The image processing system according to [12] or [13], further comprising a visualization unit that visualizes a portion of the local joint image, which is influenced in detection of the joint state by the state detection model, in a display mode corresponding to a degree of the influence.
[15] The image processing system according to any one of [11] to [14], wherein the joint state indicates normality, abnormality, bleeding, synovitis or arthrosis.
[16] The image processing system according to [15], wherein when indicating abnormality, bleeding, synovitis or arthrosis, the joint state further indicates a relevant area in the local joint image.
[17] The image processing system according to any one of [11] to [16],
   wherein the joint is a foot joint,
   the joint landmark is a tibia, a fibula, a talus or fat tissue, and
   the joint state indicates normality, abnormality, bleeding, synovitis or arthrosis.
[18] The image processing system according to any one of [11] to [16],
   wherein the joint is a knee joint,
   the joint landmark is a femur, a tibia, a fibula, a patella or fat tissue, and
   the joint state indicates normality, abnormality, bleeding, synovitis or arthrosis.
[19] The image processing system according to any one of [11] to [16],
   wherein the joint is an elbow joint,
   the joint landmark is a humerus, a radius, an ulna or fat tissue, and
   the joint state indicates normality, abnormality, bleeding, synovitis or arthrosis.
[20] The image processing system according to any one of [11] to [19], wherein diagnosis of the joint state is supported based on the joint image.
[21] An image processing program for causing a computer to function as
   a landmark detection unit that detects, in a joint image which is an in vivo image of a human joint, two or more joint landmarks, each of which is a landmark for the joint, and
   a determination unit that determines, in the joint image, a region of interest for the joint based on the two or more joint landmarks detected.
[22] The image processing program according to [21], wherein the landmark detection unit performs detection using a landmark detection model which, upon input of the joint image, detects the two or more joint landmarks in the joint image.
[23] The image processing program according to [22], wherein the landmark detection model is a model trained based on training data comprising a pair of the joint image and an image in which the two or more joint landmarks in the joint image are annotated.
[24] The image processing program according to any one of [21] to [23], wherein the landmark detection unit also detects types of the joint landmarks, and
   the determination unit performs determination based further on the detected types of the joint landmarks.
[25] The image processing program according to any one of [21] to [24], wherein one of the two or more joint landmarks detected is a bone, and another is a different bone, cartilage, fat tissue, a joint capsule or muscle.
[26] The image processing program according to any one of [21] to [25], wherein the joint is a joint of a hemophilia patient.
[27] The image processing program according to any one of [21] to [26], wherein the joint image is an ultrasound image or an X-ray image.
[28] The image processing program according to any one of [21] to [27], wherein the joint image is an image having enhanced image quality.
[29] The image processing program according to any one of [21] to [28], wherein the joint landmark is a tibia, a talus, a fibula, a femur, a patella, a humerus, a radius, an ulna, cartilage, fat tissue, a joint capsule, or muscle.
[30] The image processing program according to any one of [21] to [29], wherein the determination unit determines the region of interest based on a distance from a center of the joint landmark to a boundary line of the region of interest.
[31] The image processing program according to any one of [21] to [30], for causing the computer to function further as a state detection unit that detects a joint state which is a state of the joint, based on a local joint image which is an image of an inside of the determined region of interest in the joint image.
[32] The image processing program according to [31], wherein the state detection unit performs detection using a state detection model which, upon input of the local joint image, detects the joint state of the joint shown by the local joint image.
[33] The image processing program according to [32], wherein the state detection model is a model trained based on training data comprising a pair of the local joint image and the local joint image with which a normality label is associated or the local joint image in which an abnormality label is associated with an abnormal area of the joint shown by the local joint image.
[34] The image processing program according to [32] or [33], for causing the computer to function further as a visualization unit that visualizes a portion of the local joint image, which is influenced in detection of the joint state by the state detection model, in a display mode corresponding to a degree of the influence.
[35] The image processing program according to any one of [31] to [34], wherein the joint state indicates normality, abnormality, bleeding, synovitis or arthrosis.
[36] The image processing program according to [35], wherein when indicating abnormality, bleeding, synovitis or arthrosis, the joint state further indicates a relevant area in the local joint image.
[37] The image processing program according to any one of [31] to [36],
   wherein the joint is a foot joint,
   the joint landmark is a tibia, a fibula, a talus or fat tissue, and
   the joint state indicates normality, abnormality, bleeding, synovitis or arthrosis.
[38] The image processing program according to any one of [31] to [36],
   wherein the joint is a knee joint,
   the joint landmark is a femur, a tibia, a fibula, a patella or fat tissue, and
   the joint state indicates normality, abnormality, bleeding, synovitis or arthrosis.
[39] The image processing program according to any one of [31] to [36],
   wherein the joint is an elbow joint,
   the joint landmark is a humerus, a radius, an ulna or fat tissue, and
   the joint state indicates normality, abnormality, bleeding, synovitis or arthrosis.
[40] The image processing program according to any one of [31] to [39], wherein diagnosis of the joint state is supported based on the joint image.
[41] An image processing method executed by a computer, comprising:
   a landmark detection step of detecting, in a joint image which is an in vivo image of a human joint, two or more joint landmarks, each of which is a landmark for the joint, and
   a determination step of determining, in the joint image, a region of interest for the joint based on the two or more joint landmarks detected.
[42] An image processing device comprising:
   a landmark detection unit that detects, in a joint image which is an in vivo image of a human joint, two or more joint landmarks, each of which is a landmark for the joint; and
   a determination unit that determines, in the joint image, a region of interest for the joint based on the two or more joint landmarks detected.
[43] The image processing device according to [42], further comprising a state detection unit that detects a joint state which is a state of the joint, based on a local joint image which is an image of an inside of the determined region of interest in the joint image.

### [Advantageous Effect of Invention]

According to an aspect of the present disclosure, a more accurate region of interest can be determined.

### [Brief Description of Drawings]

[Figure 1] Figure 1 shows a diagram illustrating an example of a system configuration of an image processing system according to an embodiment.
[Figure 2] Figure 2 shows a diagram illustrating an example of a functional configuration of a preparation device according to an embodiment.
[Figure 3] Figure 3 shows a diagram illustrating an example of a hardware configuration of a computer used in a preparation device according to an embodiment.
[Figure 4] Figure 4 shows a flowchart illustrating an example of a process executed by a preparation device according to an embodiment.
[Figure 5] Figure 5 shows a flowchart illustrating another example of a process executed by a preparation device according to an embodiment.
[Figure 6] Figure 6 illustrates an example of a training ankle image prepared by the process shown in Figure 5.
[Figure 7] Figure 7 shows a diagram illustrating a configuration of a preparation program according to an embodiment.
[Figure 8] Figure 8 shows a diagram illustrating an example of a functional configuration of a training device according to an embodiment.
[Figure 9] Figure 9 shows a diagram illustrating an example of a hardware configuration of a computer used in a training device according to an embodiment.
[Figure 10] Figure 10 shows a diagram illustrating an example of determination of ROI1.
[Figure 11] Figure 11 shows a flowchart illustrating an example of a process executed by a training device according to an embodiment.
[Figure 12] Figure 12 shows a flowchart illustrating another example of a process executed by a training device according to an embodiment.
[Figure 13] Figure 13 illustrates an example of a training ankle image prepared by the process shown in Figure 12.
[Figure 14] Figure 14 shows a diagram illustrating a configuration of a training program according to an embodiment.
[Figure 15] Figure 15 shows a diagram illustrating an example of a functional configuration of an evaluation device according to an embodiment.
[Figure 16] Figure 16 shows a diagram illustrating an example of a hardware configuration of a computer used in an evaluation device according to an embodiment.
[Figure 17] Figure 17 shows a flowchart illustrating an example of a process executed by an evaluation device according to an embodiment.
[Figure 18] Figure 18 shows a flowchart illustrating another example of a process executed by an evaluation device according to an embodiment.
[Figure 19] Figure 19 illustrates an example of a training ankle image prepared by the process shown in Figure 18.
[Figure 20] Figure 20 shows a diagram illustrating an example of a joint landmark of an ankle image.
[Figure 21] Figure 21 shows a diagram illustrating an example of a joint landmark of a knee image.
[Figure 22] Figure 22 shows a diagram illustrating another example of a joint landmark of a knee image.
[Figure 23] Figure 23 shows a diagram illustrating an example of a joint landmark of an elbow image.
[Figure 24] Figure 24 shows a diagram illustrating another example of a joint landmark of an elbow image.
[Figure 25] Figure 25 shows a diagram illustrating a configuration of an evaluation program according to an embodiment.
[Figure 26] Figure 26 shows a diagram illustrating an example of a functional configuration of a display device according to an embodiment.
[Figure 27] Figure 27 shows a diagram illustrating an example of a hardware configuration of a computer used in a display device according to an embodiment.
[Figure 28] Figure 28 shows a diagram illustrating a configuration of a display program according to an embodiment.

### [Description of Embodiments]

In the following, embodiments of the present disclosure will be described in detail with reference to drawings. In the description of the drawings, identical elements are provided with the same reference sign, and overlapping description is omitted. Embodiments of the present disclosure in the following description are specific examples of the present invention, and should not be construed as limiting the present invention unless it is indicated that the present invention is limited to these embodiment.

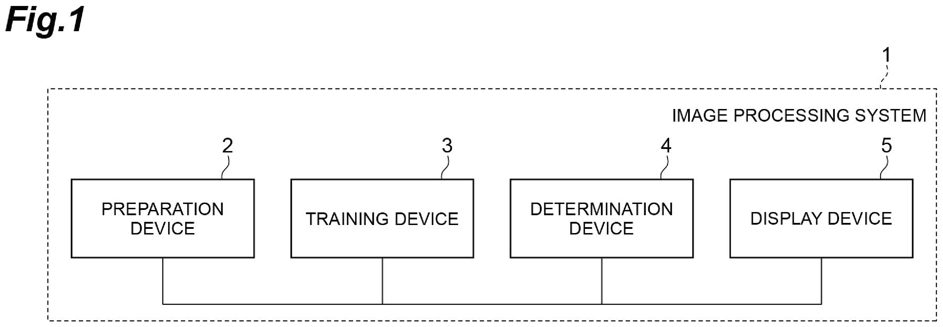

Figure 1 shows a diagram illustrating an example of a system configuration of an image processing system 1 (image processing system) according to an embodiment. As shown in Figure 1, the image processing system 1 comprises a preparation device 2 according to an embodiment, a training device 3 according to an embodiment, an evaluation device 4 (image processing device) according to an embodiment, and a display device 5 according to an embodiment. The image processing system 1 may further comprise other arbitrary devices. A network such as LAN (Local Area Network) or Internet provides communication connections of the devices in the image processing system 1, so that information can be transmitted and received among the devices. Two or more devices in the image processing system 1 may configured as one device having all of the functions of the two or more devices.

### [Preparation device 2]

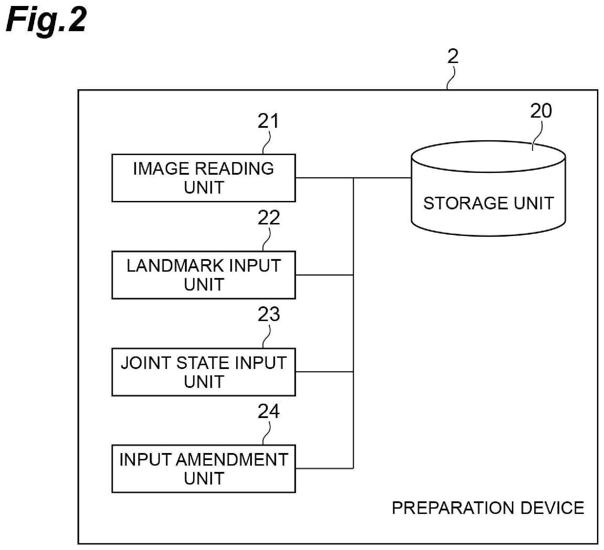

The preparation device 2 is a computer device that prepares teacher data. Figure 2 shows a diagram illustrating an example of a functional configuration of the preparation device 2. As shown in Figure 2, the preparation device 2 comprises a storage unit 20, an image reading unit 21, a landmark input unit 22, a joint state input unit 23, and an input amendment unit 24.

The functional blocks of the preparation device 2 are assumed to function in the preparation device 2, but the present invention is not limited thereto. For example, a part of the functional blocks of the preparation device 2 may be a computer device different from the preparation device 2, which functions while transmitting and receiving information between itself and the preparation device 2 in a computer device (for example, the training device 3, the evaluation device 4 or the display device 5) that is connected through a network to the preparation device 2. A part of the functional blocks of the preparation device 2 may be absent, a plurality of functional blocks may be integrated into one functional block, or one functional block may be divided into a plurality of functional blocks.

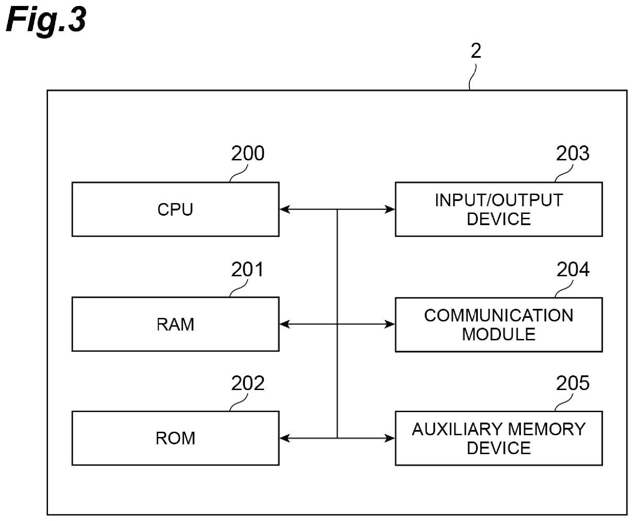

Figure 3 shows a diagram illustrating an example of a hardware configuration of a computer used in the preparation device 2. Physically, the preparation device 2 is configured as a computer system comprising a CPU (central processing unit) 200 which is a central processor (processor), a RAM (random access memory) 201 and a ROM (read only memory) 202 which are main memory devices, an input/output device 203 such as a keyboard, a microphone or a display, a communication module 204 which is a data transmitting and receiving device, and an auxiliary memory device 205 such as a hard disk or a SSD (solid state drive), as shown in Figure 3. There may be a plurality of CPUs 200, RAMs 201, ROMs 202, input/output devices 203, communication modules 204 and auxiliary memory devices 205. The functions of the functional blocks shown in Figure 2 are performed by causing predetermined computer software to be read on hardware such as the CPU 200 and the RAM 201 shown in Figure 3, thereby operating the input/output device 203 and the communication module 204 under control of the CPU 200, and reading and writing data in the RAM 201 and the auxiliary memory device 205.

Hereinafter, the functions of the preparation device 2 shown in Figure 2 will be described.

The storage unit 20 stores arbitrary information used or output in, for example, a process in the preparation device 2. The storage unit 20 may store information calculated by the functions of the preparation device 2. The information stored by the storage unit 20 may be appropriately referred to by the functions of the preparation device 2, or may be appropriately referred to via a network by the functions of the training device 3, the evaluation device 4 or the display device 5.

The image reading unit 21 reads a joint image which is an in vivo image of a human joint (ankle, elbow, knee or the like). The image reading unit 21 may read a joint image using the input/output device 203, or may read a joint image via a network from another device or the like using the communication module 204. For example, the image reading unit 21 may read a joint image from a joint image storage module (a module for storing a joint image) of another device. The image reading unit 21 may adjust the image quality of the joint image by, for example, performing image quality enhancement (gradation adjustment, noise removal or the like) on the joint image in reading of the joint image. The image reading unit 21 may allow the read joint image to be stored by the storage unit 20, or output the read joint image to another functional block of the preparation device 2.

In the present embodiment, the "joint" may be a joint of a coagulation defect patient or a rheumatoid arthritis patient, and is preferably a joint of hemophilia patient. In the present embodiment, the "joint image" may be an ultrasound image or an X-ray image. In the present embodiment, the "joint image" may be an image having enhanced image quality.

The landmark input unit 22 accepts input for a joint landmark, which is a landmark for a joint, with respect to a joint image. The landmark input unit 22 may accept input for a joint landmark with respect to a joint image read by the image reading unit 21. More specifically, the landmark input unit 22 may accept input for a joint landmark with respect to a joint image read by the image reading unit 21 and stored by the storage unit 20, or may accept input for a joint landmark with respect to a joint image read by the image reading unit 21 and output to the landmark input unit 22 from the image reading unit 21.

The joint landmark, when in, for example, a joint image, is a characteristic area (in the context of position, shape, pattern, luminance, brightness or the like) of an anatomical tissue/organ in a joint on the joint image. The joint landmark may be a tibia, a talus, a fibula, a femur, a patella, a humerus, a radius, an ulna, cartilage, fat tissue, a joint capsule, or muscle. How joint landmarks, synovitis and bleeding look in a joint image is disclosed in, for example, the following References 1 to 4.
Reference 1: Journal of the Kyorin Medical Society, Vol. 48, No. 1, 67-73, March, 2017 https://www.jstage.jst.go.jp/article/kyorinmed/48/1/48_67/_pdf
Reference 2: "Hazimete No Seikei Geka Choonpa Kensa" (Introduction to Orthopedic Ultrasound Examination) https://www.konicaminolta.jp/healthcare/products/us/snible2/pdf/snible_dr_minagawa.pdf
Reference 3: Choonpa Kensa Gijutsu (Ultrasound Examination Technology) (1881-4506), Vol. 47, No. 2, Page 153-157 (2022. 04), DOI : 10.11272/jss. r549
Reference 4: Shoni Naika (Pediatric Internal Medicine) (0385-6305), Vol. 54, Extra Edition, Page 671-676 (2022. 12)

The landmark input unit 22 may accept input for a joint landmark from a user of the preparation device 2 (a physician or the like). More specifically, the landmark input unit 22 may display a joint image to a user of the preparation device 2 using the input/output device 203, and accept annotation and labeling of (one or more) landmarks by the user with respect to the joint image. The annotation means that a line is drawn along the joint landmark or a line is drawn so as to surround the joint landmark on a joint image, for example. The labeling means that a type of the joint landmark is associated with (for example, a color corresponding to a type of the landmark is given to) each of the lines drawn by annotation, for example. The landmark input unit 22 may accept annotation and labeling of two or more joint landmarks (including labeling of different landmarks in different ways) by a user of the preparation device 2. The type of the joint landmark may be a tibia, a talus, a fibula, a femur, a patella, a humerus, a radius, an ulna, cartilage, fat tissue, a joint capsule, or muscle as described above.

The landmark input unit 22 may add landmark input information, which is information on input for the accepted joint landmark, to a joint image, and then allow the joint image to be stored by the storage unit 20, or output the joint image to another functional block of the preparation device 2.

The joint state input unit 23 accepts input for a joint state which is a state of a joint.

The joint state input unit 23 may accept input for a joint state with respect to a joint image read by the image reading unit 21. More specifically, the joint state input unit 23 may accept input for the joint state input unit 23 with respect to a joint image read by the image reading unit 21 and stored by the storage unit 20, or may accept input for a joint state with respect to a joint image read by the image reading unit 21 and output to the joint state input unit 23 from the image reading unit 21.

The joint state input unit 23 may accept input for a joint state with respect to a joint image reflecting landmark input information obtained by the landmark input unit 22 (for example, a joint image in which the joint landmark is annotated and labeled). More specifically, the joint state input unit 23 may reflect, based on a joint image which is stored by the storage unit 20 and to which landmark input information is added, the landmark input information on the joint image, and accept input for a joint state with respect to the reflection joint image, or may reflect, based on a joint image which is output to the joint state input unit 23 from the landmark input unit 22 and to which landmark input information is added, the landmark input information on the joint image, and accept input for a joint state with respect to the reflection joint image.

The joint state input unit 23 may indicate that the joint state is normal, abnormal, bleeding, synovitis or arthrosis. When indicating that the joint state is abnormal, bleeding, synovitis or arthrosis, the joint state input unit 23 may further indicate a relevant area in a joint image.

The joint state input unit 23 may accept input for a joint state from a user of the preparation device 2 (a physician or the like). More specifically, the joint state input unit 23 may display a joint image (which reflects or does not reflect landmark input information) to a user of the preparation device 2 using the input/output device 203, and accept labeling and annotation of a joint state by the user with respect to the joint image. The labeling means that a normality label indicating normality or an abnormality label indicating abnormality is attached to a joint image, or an abnormality label indicating abnormality is attached to an abnormal area, for example. The annotation means that a line is drawn on a joint image with an abnormal area or a joint image provided with an abnormality label such that the line extends along the abnormal area, or the line surrounds the abnormal area. The joint state input unit 23 may accept annotation of two or more joint states by a user of the preparation device 2.

The joint state input unit 23 may add joint state input information, which is information on input for the accepted joint state, to a joint image, and then allow the joint image to be stored by the storage unit 20, or output the joint image to another functional block of the preparation device 2.

The input amendment unit 24 accepts amendment of input for a joint landmark and amendment of input for a joint state with respect to a joint image. More specifically, the input amendment unit 24 accepts amendment of input for a joint landmark in a joint image reflecting landmark input information, and accepts amendment of input for a joint state in a joint image reflecting joint state input information. The input amendment unit 24 may accept amendment of input for a joint landmark from a user different from a user of the preparation device 2 who has been involved in the input. The input amendment unit 24 may accept amendment of input for a joint state from a user different from a user of the preparation device 2 who has been involved in the input.

The input amendment unit 24 may add the amended landmark input information to a joint image, and then allow the joint image to be stored by the storage unit 20, or output the joint image to another functional block of the preparation device 2. The input amendment unit 24 may add the amended joint state input information to the joint image, and then allow the joint image to be stored by the storage unit 20, or output the joint image to another functional block of the preparation device 2.

The joint image treated by the preparation device 2 is data that is finally used in training by the training device 3 (teaching data). Therefore, the joint image treated by the preparation device 2 is referred to as a training joint image as appropriate.

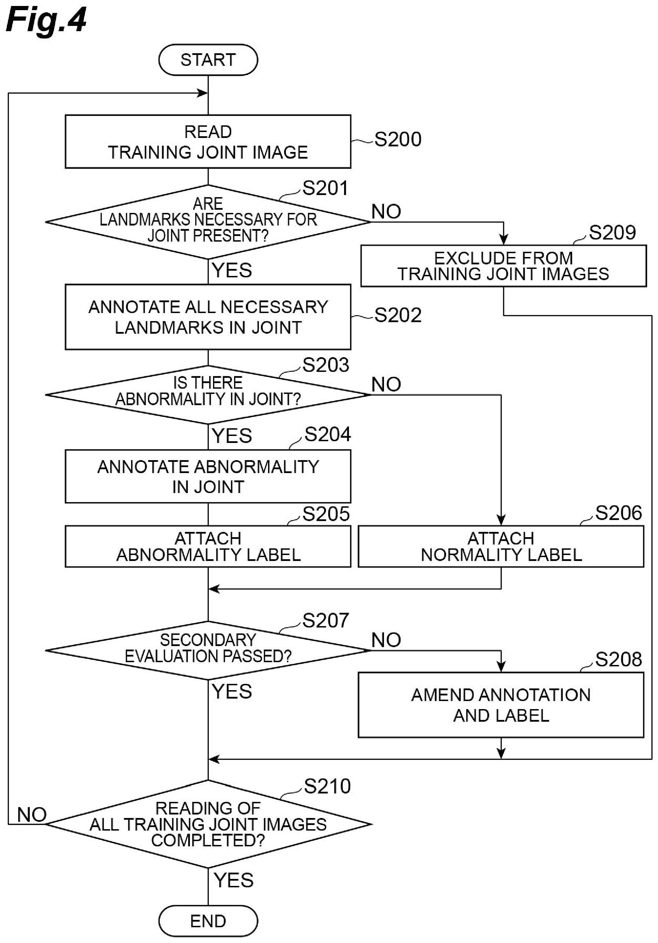

Figure 4 shows a flowchart illustrating an example of a process executed by a preparation device. More specifically, Figure 4 shows a flowchart illustrating a process for adding landmark input information and joint state input information to a training joint image.

First, the image reading unit 21 reads a training joint image (step S200). Next, a user A, a user of the preparation device 2, determines whether or not the training joint image (displayed by the preparation device 2) has landmarks necessary for a joint (step S201). When it is determined in step S201 that the image has the landmarks (S201: YES), the user A annotates (and labels) all the necessary landmarks in the joint in the training joint image, and the landmark input unit 22 accepts the relevant input (step S202). Next, the user A determines whether or not the joint has an abnormality in the training joint image (step S203).

When it is determined in step S203 that the joint has an abnormality (S203: YES), the user A annotates the abnormality in the joint in the training joint image, and the joint state input unit 23 accepts the relevant input (step S204). Next, the user A attaches an abnormality label in the training joint image, and the joint state input unit 23 accepts the relevant input (step S205). When it is determined in step S203 that the joint has no abnormality (S203: NO), the user A attaches a normality label in the training joint image, and the joint state input unit 23 accepts the relevant input (step S206).

Subsequent to step S205 or step S206, a user B, a user of the preparation device 2 that is different from the user A, performs secondary evaluation in which the previous input by the user A is evaluated, followed by determination of pass or not in the secondary evaluation (step S207). When determination of failure to pass is made in step S207 (S207: NO), the user B amends wrong portions of the previous annotation and/or labeling by the user A, and the input amendment unit 24 accepts the amendment (step S208).

When it is determined in step S201 that the image does not have the landmarks (S201: NO), the user A excludes the joint image, which has been read in step S200, from the training joint images (step S209). Subsequent to step S208 or step S209, or when determination of pass is made in step S207 (S207: YES), the preparation device 2 determines whether or not reading of all the training joint images has been completed (step S210). When it is determined in step S210 that the reading has been completed (step S210: YES), the process is terminated. When it is determined in step S210 that the reading has not been completed (S210: NO), the processes of step S200 and the subsequent steps are repeated for the remaining training joint images.

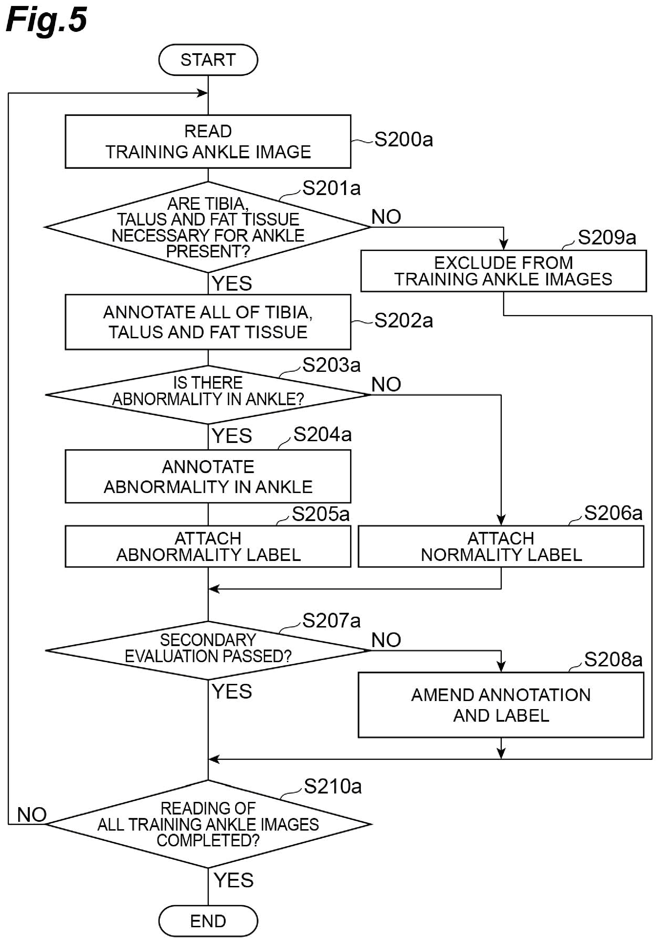

Figure 5 shows a flowchart illustrating an example of a process executed by a preparation device. More specifically, Figure 5 shows, with respect the flowchart shown in Figure 4, a specific example in which bleeding in an ankle is evaluated. For example, in Figure 5, the training joint image in Figure 4 is replaced by a training ankle image focused on an ankle. When the joint is a foot joint, a tibia, a talus and fat tissue may be used as landmarks.

First, the image reading unit 21 reads a training ankle image (step S200a). Next, a user A, a user of the preparation device 2, determines whether or not the training ankle image (displayed by the preparation device 2) has a tibia, a talus and fat tissue necessary for an ankle (step S201a). When it is determined in step S201a that the image has the landmarks (S201a: YES), the user A annotates (and labels) all of the tibia, the talus and the fat tissue in the training ankle image, and the landmark input unit 22 accepts the relevant input (step S202a). Next, the user A determines whether or not the ankle has an abnormality in the training ankle image (step S203a).

When it is determined in step S203a that the joint has an abnormality (S203a: YES), the user A annotates the abnormality in the ankle in the training ankle image, and the joint state input unit 23 accepts the relevant input (step S204a). Next, the user A attaches an abnormality label in the training ankle image, and the joint state input unit 23 accepts the relevant input (step S205). When it is determined in step S203a that the joint has no abnormality (S203a: NO), the user A attaches a normality label in the training ankle image, and the joint state input unit 23 accepts the relevant input (step S206a).

Subsequent to step S205a or step S206a, a user B, a user of the preparation device 2 that is different from the user A, performs secondary evaluation in which the previous input by the user A is evaluated, followed by determination of pass or not in the secondary evaluation (step S207a). When determination of failure to pass is made in step S207a (S207a: NO), the user B amends wrong portions of the previous annotation and/or labeling by the user A, and the input amendment unit 24 accepts the amendment (step S208a).

When it is determined in step S201a that the image does not have the landmarks (S201a: NO), the user A excludes the ankle image, which has been read in step S200a, from the training ankle images (step S209a). Subsequent to step S208a or step S209a, or when determination of pass is made in step S207a (S207a: YES), the preparation device 2 determines whether or not reading of all the training ankle images has been completed (step S210a). When it is determined in step S210a that the reading has been completed (step S210a: YES), the process is terminated. When it is determined in step S210a that the reading has not been completed (S210a: NO), the processes of step S200a and the subsequent steps are repeated for the remaining training ankle images.

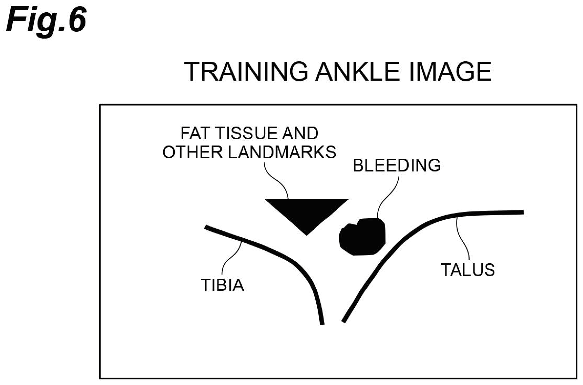

Figure 6 illustrates an example of a training ankle image which is prepared by the process shown in Figure 5. In the training ankle image shown in Figure 6, the tibia, the talus and the fat tissue annotated (and labeled) in step S202a of Figure 5, and bleeding annotated in step S204a of Figure 5 are shown.

Hereinafter, a training joint image to which at least one of landmark input information and joint state input information is added by the preparation device 2 is referred to simply as a training joint image.

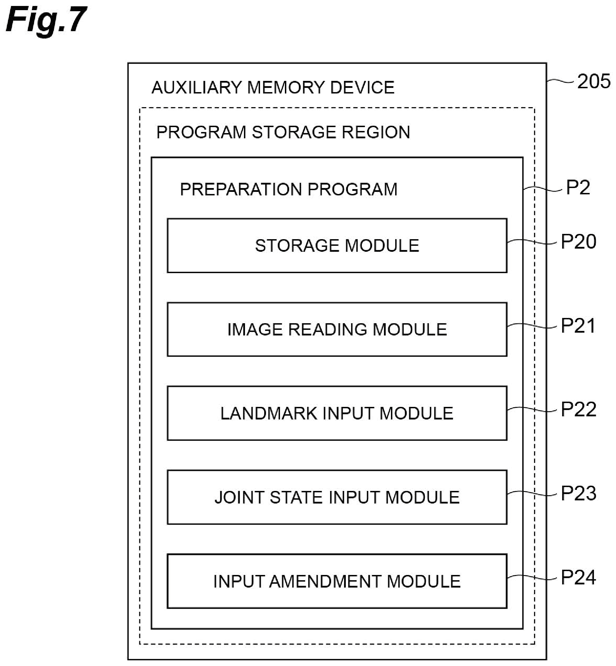

Next, a preparation program P2 for causing a computer to execute a series of processes by the preparation device 2 will be described. For example, as shown in Figure 7, the preparation program P2 is stored in a program storage region formed in the auxiliary memory device 205 of the preparation device 2.

The preparation program P2 comprises a storage module P20, an image reading module P21, a landmark input module P22, a joint state input module P23, and an input amendment module P24. Functions that are performed by executing the storage module P20, the image reading module P21, the landmark input module P22, the joint state input module P23 and the input amendment module P24 are similar, respectively, to the functions of the storage unit 20, the image reading unit 21, the landmark input unit 22, the function state input unit 23 and the input amendment unit 24 of the preparation device 2. The preparation program P2 is a program for causing the preparation device 2 (one or more CPUs thereof) to function as the storage unit 20, the image reading unit 21, the landmark input unit 22, the joint state input unit 23 and the input amendment unit 24.

A part or the whole of the preparation program P2 may be transmitted via a transmission medium such as a communication line, and received by another equipment, followed by being stored (as well as being installed). The modules of the preparation program P2 may be installed not in one computer, but in any of a plurality of computers. In this case, the series of processes in the preparation program P2 is carried out by the computer systems of the plurality of computers.

### [Training device 3]

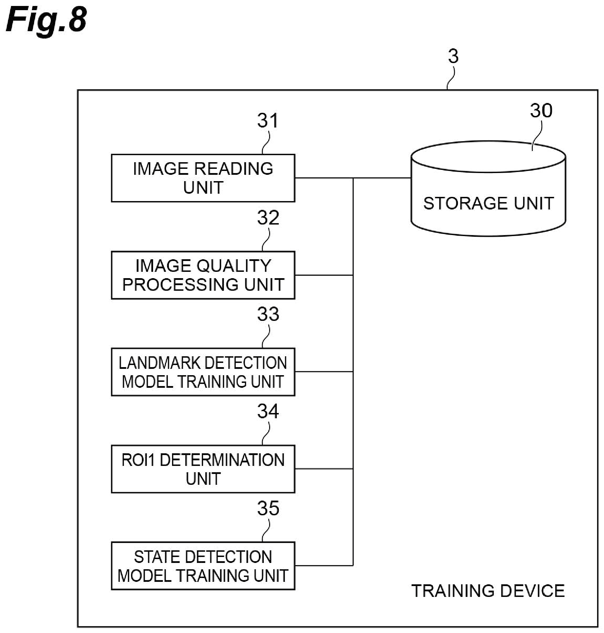

The training device 3 is a computer device that trains various detection models based on training joint images which are teacher data prepared by the preparation device 2. Figure 8 shows a diagram illustrating an example of a functional configuration of the training device 3. As shown in Figure 8, the training device 3 comprises a storage unit 30, an image reading unit 31, an image quality processing unit 32, a landmark detection model training unit 33, a ROI1 determination unit 34, and a state detection model training unit 35.

The functional blocks of the training device 3 are assumed to function in the training device 3, but the present invention is not limited thereto. For example, a part of the functional blocks of the training device 3 may be a computer device different from the training device 3, and function while appropriately sending and receiving information between itself and the training device 3 in a computer device (for example, the preparation device 2, the evaluation device 4 or the display device 5) that is network-connected to the training device 3. A part of the functional blocks of the training device 3 may be absent, a plurality of functional blocks may be integrated into one functional block, or one functional block may be divided into a plurality of functional blocks.

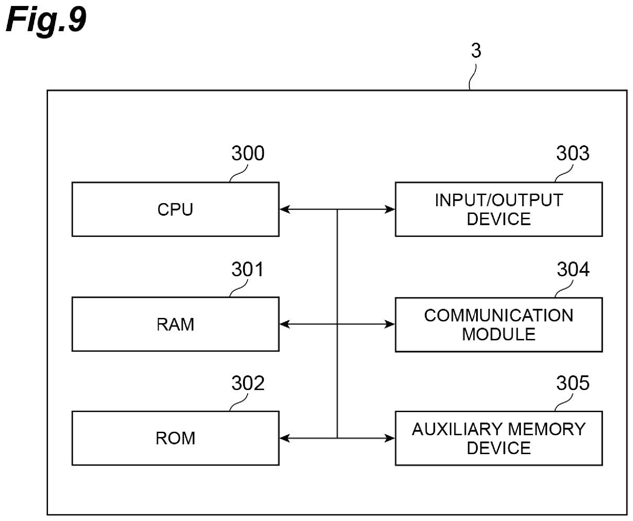

Figure 9 shows a diagram illustrating an example of a hardware configuration of a computer used in the training device 3. Physically, the training device 3 is configured as a computer system comprising a CPU 300, a RAM 301 and a ROM 302, an input/output device 303 such as a keyboard, a microphone or a display, a communication module 304 which is a data transmitting and receiving device, and an auxiliary memory device 305 such as a hard disk or a SSD, as shown in Figure 9. There may be a plurality of CPUs 300, RAMs 301, ROMs 302, input/output devices 303, communication modules 304 and auxiliary memory devices 305. The functions of the functional blocks shown in Figure 8 are performed by causing predetermined computer software to be read on hardware such as the CPU 300 and the RAM 301 shown in Figure 9, thereby operating the input/output device 303 and the communication module 304 under control of the CPU 300, and reading and writing data in the RAM 301 and the auxiliary memory device 305.

Hereinafter, the functions of the training device 3 shown in Figure 8 will be described.

The storage unit 30 stores arbitrary information used or output in, for example, a process in the training device 3. The storage unit 30 may store information calculated by the functions of the training device 3. The information stored by the storage unit 30 may be appropriately referred to by the functions of the training device 3, or may be appropriately referred to via a network by the functions of the preparation device 2, the evaluation device 4 or the display device 5.

The image reading unit 31 reads a training joint image. The image reading unit 31 may read a training joint image using the input/output device 303, or may read a training joint image via a network from another device or the like using the communication module 304. For example, the image reading unit 31 may read a training joint image (to which landmark input information and joint state input information are added by the preparation device 2) from the storage unit 20 of the preparation device 2. The image reading unit 31 may allow the read training joint image to be stored by the storage unit 30, or output the read joint image to another functional block of the training device 3.

The image quality processing unit 32 may adjust the image quality of a training joint image by, for example, performing image quality enhancement (gradation adjustment, noise removal or the like) on the training joint image. The image quality processing unit 32 may adjust the image quality of a training joint image read by the image reading unit 31. More specifically, the image quality processing unit 32 may adjust the image quality of a training joint image read by the image reading unit 31 and stored by the storage unit 30, or may adjust the image quality of a training joint image read by the image reading unit 31 and output to the image quality processing unit 32 from the image reading unit 31. The image quality processing unit 32 may allow the training joint image subjected to adjustment of image quality to be stored by the storage unit 30, or output the training joint image to another functional block of the training device 3.

The landmark detection model training unit 33 trains using a training joint image, and generates a landmark detection model. The landmark detection model training unit 33 may train using a training joint image read by the image reading unit 31, or may train using a training joint image subjected to adjustment of image quality by the image quality processing unit 32. More specifically, the landmark detection model training unit 33 may train using a training joint image read by the image reading unit 31 and stored by the storage unit 30, may train using a training joint image read by the image reading unit 31 and output to the landmark detection model training unit 33 from the image reading unit 31, may train using a training joint image subjected to adjustment of image quality by the image quality processing unit 32 and stored by the storage unit 30, or may train using a training joint image subjected to adjustment of image quality by the image quality processing unit 32 and output to the landmark detection model training unit 33 from the image quality processing unit 32.

The landmark detection model is a trained model which, upon input of a joint image, detects a joint landmark in the joint image. The landmark detection model may be a model trained based on training data comprising a pair of a joint image and an image in which a joint landmark in the joint image is annotated. For example, the landmark detection model training unit 33 trains based on training data comprising a pair of a training joint image which does not reflect landmark input information and a training joint image which reflects landmark input information, followed by generation of a landmark detection model.

The landmark detection model may be a trained model which, upon input of a joint image, detects two or more joint landmarks in the joint image. The landmark detection model may be a model trained based on training data comprising a pair of a joint image and an image in which two or more joint landmarks in the joint image are annotated. For example, the landmark detection model training unit 33 trains based on training data comprising a pair of a training joint image which does not reflect landmark input information that includes annotations of two or more joint landmarks and a training joint image which reflects the landmark input information (that includes annotations of two or more joint landmarks), followed by generation of a landmark detection model.

The landmark detection model training unit 33 may allow the landmark detection model to be trained for each joint.

The ROI1 determination unit 34 detects a joint landmark in a training joint image, and determines (detects, identifies or cuts out) ROI1 which is a region of interest (ROI) based on the position of the detected joint landmark. The ROI1 determination unit 34 may detect a joint landmark based on landmark input information added to the training joint image, or may detect a joint landmark using a landmark detection model generated by the landmark detection model training unit 33. The ROI1 determination unit 34 may determine ROI1 based on two or more joint landmarks detected. The ROI1 determination unit 34 may determine ROI1 based further on the types of the joint landmarks detected.

The ROI1 determination unit 34 may determine ROI1 based on the offset of the detected joint landmark. More specifically, the ROI1 determination unit 34 may determine the offset of the periphery based on the label and the relative position of the detected joint landmark, and determine ROI1. The ROI1 determination unit 34 may determine ROI1 based on the distance from the center of the detected joint landmark to the boundary line of ROI1.

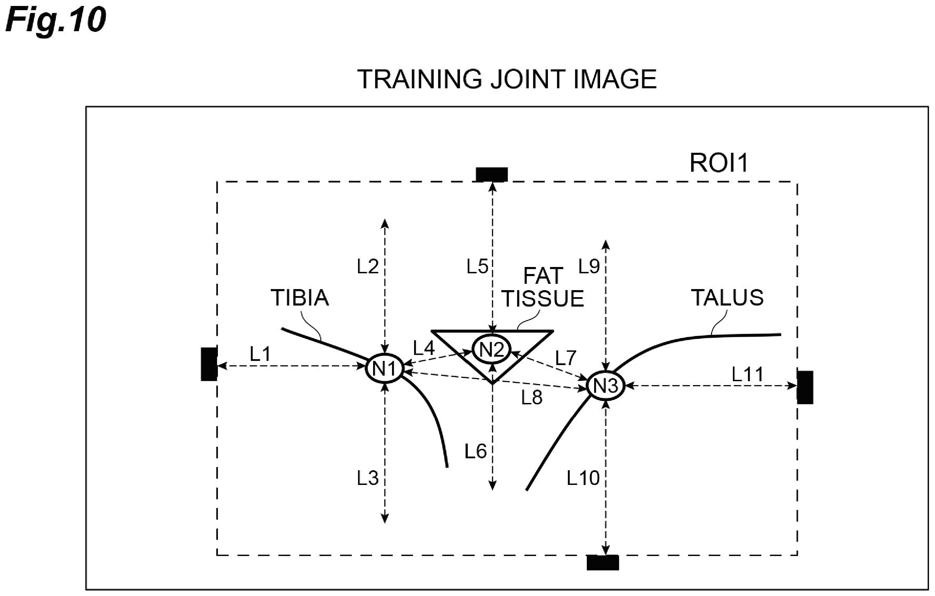

Figure 10 shows a diagram illustrating an example of determination of ROI1. A method for determining ROI1 (ankle joint ROI) by the labels and the relative positions of two or more joint landmarks will be described using a tibia, a talus and fat tissue in an ankle as examples of joint landmarks as shown in Figure 10. First, node Nos. 1, 2 and 3 (hereinafter, described as N1, N2 and N3) are assigned to the centers (center positions of lines or geometric centers) of the tibia, the talus and the fat tissue. Next, distances from the nodes (joint landmarks) to other nodes (L4, L7 and L8) are measured to know positional relationships between the joint landmarks. Next, offset distances from the nodes (joint landmarks) to the four peripheries are measured, boundary lines of ROI1 are determined by the uppermost, lowermost, leftmost and rightmost positions on coordinates (L1, L5, L11 and L10), and ROI is determined. The larger the number of joint landmarks, the more accurate the estimation of the relative positions and the determination of ROI1.

The term "offset distance" refers to distances between the centers of joint landmarks (N1, N2 and N3 in Figure 10) and the boundary lines of ROI1 (example: L1, L5, L11 and L10 in Figure 10), and distances between joint landmarks (example: L4, L7 and L8). The specific value of the offset distance may be determined from a clinical (human joint structure) point of view. For example, the boundary line of ROI1 in Figure 10 can be determined by setting offset distances from the centers of the joint landmarks (N1, N2 and N3 in Figure 10) so as to appropriately display the joint (abnormality of the joint and joint landmarks) in ROI1. For example, in Figure 10, the boundary lines of ROI1 are determined based on a leftward offset distance from N1 that is positioned on the leftmost side, an upward offset distance from N2 which is positioned on the uppermost side, and rightward and downward offset distances from N3 which is positioned on the rightmost and lowermost side. From a clinical point of view, the offset distance may be set as a distance which ensures that the region of a joint does not appear outside the ROI1. The offset distance may vary between adults and children, between males and females and between races (Asians and Westerners). For example, for children, females and Asians, whose joint ROI1 is smaller than that of adults, males and Westerners, respectively, the corresponding offset distance may be accordingly reduced. When the offset distance varies, offset distances specific to an adult, a child, a male, a female and each of the races are artificially input to the training device 3 or the evaluation device 4, whereby ROI1 corresponding to an offset distance specific to each of the types can be cut out. Using a trained model trained based on joint images labeled with joint image offset distances specific to an adult, a child, a male, a female and each of the races, ROI1 corresponding to the offset distance specific to each of the types may be cut out.

ROI1 is determined by the labels and the relative positions of joint landmarks as described above. The type of the joint landmark is identified by the label. For example, the left-side boundary line of ROI1 is determined by the L1 offset distance of the "tibia" (identified by the label). Thus, when the label of the joint landmark is known, for example, the left-side boundary line of ROI1 can be identified. If some joint landmarks are not detected, the relative position can be used to estimate the joint landmarks. In Figure 10, a tibia, a talus and fat tissue of an ankle are detected. If any of them is undetected, the undetected joint landmark can be estimated based on the labels (types) and the relative positions of detected joint landmarks. For example, in the case where the talus is undetected, it can be estimated that the intersection of two circles at distance L8 from the tibia and at distance L7 from the fat tissue is the talus. In this way, an undetected joint landmark can be estimated by the relative position. In this way, a more accurate range of ROI1 can be identified.

The ROI1 determination unit 34 may allow ROI1 information, which is information on determined ROI1, to be stored by the storage unit 30, or output the ROI1 information to another functional block of the training device 3.

The state detection model training unit 35 trains using a local joint image which is an image of the inside of ROI1 determined by the ROI1 determination unit 34 among training joint images, followed by generation of a state detection model. The training joint image is similar to the training joint image used by the landmark detection model training unit 33. The local joint image may be an image of the inside of ROI1 shown by ROI1 information stored by the storage unit 30, or an image of the inside of ROI1 shown by ROI1 information output to the state detection model training unit 35 from the ROI1 determination unit 34, in the training joint image.

The state detection model is a trained model which upon input of a local joint image, detects a joint state of a joint shown by the local joint image. The state detection model may be a model trained based on training data comprising a pair of a local joint image and the local joint image with which a normality label is associated or the local joint image in which an abnormality label is associated with an abnormal area of the joint shown by the local joint image. For example, the state detection model training unit 35 trains based on training data comprising a pair of a local joint image in a training joint image which does not reflect joint state input information and a local joint image in a training joint image which reflects joint state input information, followed by generation of a state detection model.

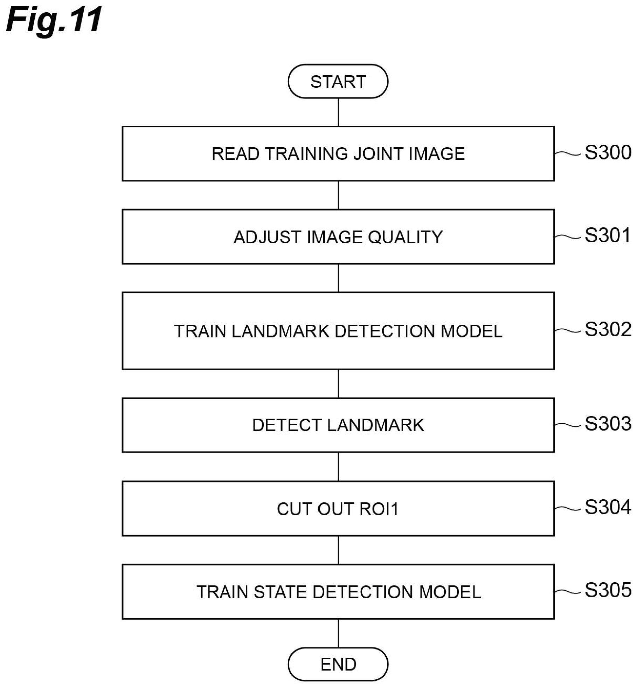

Figure 11 shows a flowchart illustrating an example of a process executed by the training device 3. More specifically, Figure 11 shows a flowchart illustrating a process for training a landmark detection model and a state detection model based on a training joint image.

First, the image reading unit 31 reads a training joint image (step S300). Next, the image quality processing unit 32 adjusts the image quality of the training joint image read in step S300 (step S301). Step S301 may be omitted. Next, the landmark detection model training unit 33 trains using the training joint image subjected to adjustment of image quality in step S301 (the training joint image read in step S300 when step S301 is omitted; the same applies hereinafter), and generates a landmark detection model (step S302). Next, the ROI1 determination unit 34 detects a joint landmark in the training joint image subjected to adjustment of image quality in step S301 (step S303), and cut outs (determines) ROI1 based on the position of the detected joint landmark (step S304). Next, the state detection model training unit 35 trains using a local joint image of the inside of ROI1 cut out in step S304 in the training joint image subjected to adjustment of image quality in step S301, followed by generation of a state detection model (step S305).

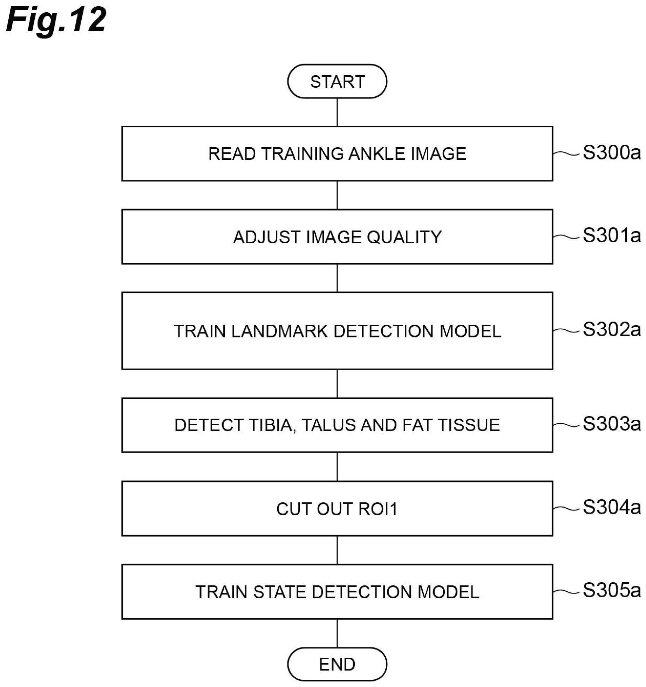

Figure 12 shows a flowchart illustrating another example of a process executed by the training device 3. More specifically, Figure 12 shows, with respect to the flowchart shown in Figure 11, a specific example in which various detection models for evaluating bleeding in an ankle are trained. For example, in Figure 12, the training joint image in Figure 11 is replaced by a training ankle image focused on an ankle. When the joint is an ankle, a tibia, a talus and fat tissue may be used as landmarks.

First, the image reading unit 31 reads a training ankle image (step S300a). Next, the image quality processing unit 32 adjusts the image quality of the training ankle image read in step S300a (step S301a). Step S301a may be omitted. Next, the landmark detection model training unit 33 trains using the training ankle image subjected to adjustment of image quality in step S301a (the training ankle image read in step S300a when step S301a is omitted; the same applies hereinafter), and generates a landmark detection model (step S302a). Next, the ROI1 determination unit 34 detects a tibia, a talus and fat tissue in the training ankle image subjected to adjustment of image quality in step S301a (step S303a), and cuts out (determines) ROI1 based on the position of each of the detected tibia, talus and fat tissue (ankle local ROI) (step 304a). Next, the state detection model training unit 35 trains using a local joint image of the inside of ROI1 cut out in step S304a in the training ankle image subjected to adjustment of image quality in step S301a, followed by generation of a state detection model (step S305a).

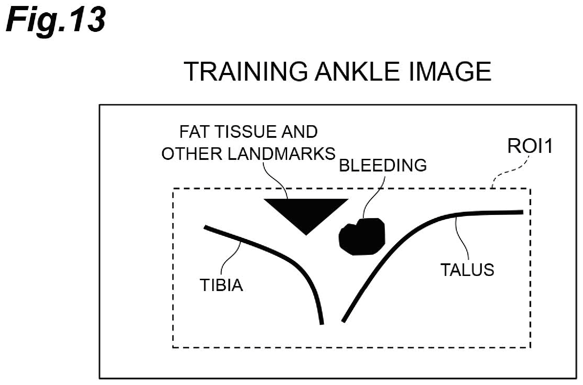

Figure 13 illustrates an example of a training ankle image which is prepared by the process shown in Figure 12. In the training ankle image shown in Figure 13, ROI1 cut out in step S304a of Figure 12 is shown.

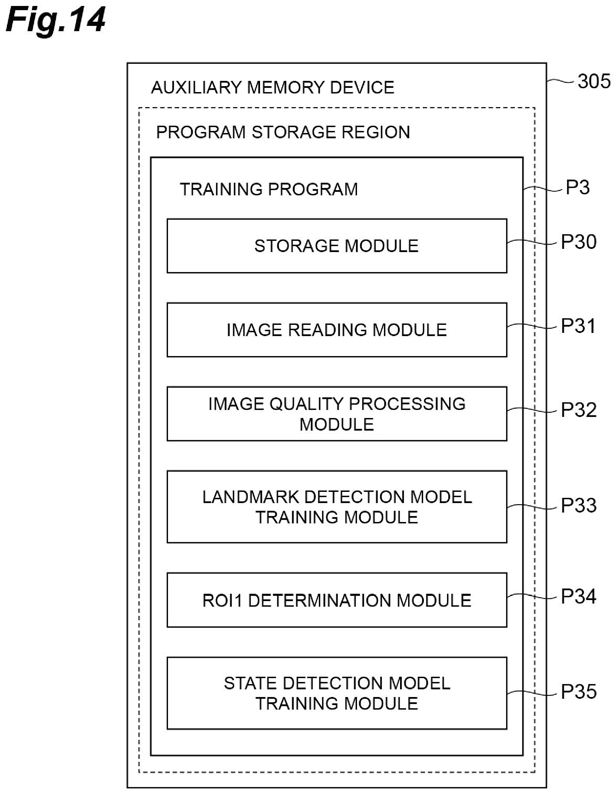

Next, a training program P3 for causing a computer to execute a series of processes by the training device 3 will be described. For example, as shown in Figure 14, the training program P3 is stored in a program storage region formed in the auxiliary memory device 305 of the training device 3.

The training program P3 comprises a storage module P30, an image reading module P31, an image quality processing module P32, a landmark detection model training module P33, a ROI1 determination module P34, and a state detection model training module P35. Functions that are performed by executing the storage module P30, the image reading module P31, the image quality processing module P32, the landmark detection model training module P33, the ROI1 determination module P34, and the state detection model training module P35 are similar, respectively, to the functions of the storage unit 30, the image reading unit 31, the image quality processing unit 32, the landmark detection model training unit 33, the ROI1 determination unit 34, and the state detection model training unit 35 of the training device 3. The training program P3 is a program for causing the training device 3 (one or more CPUs thereof) to function as the storage unit 30, the image reading unit 31, the image quality processing unit 32, the landmark detection model training unit 33, the ROI1 determination 34, and the state detection model training unit 35.

A part or the whole of the training device 3 may be transmitted via a transmission medium such as a communication line, and received by another equipment, followed by being stored (as well as being installed). The modules of the training device 3 may be installed not in one computer, but in any of a plurality of computers. In this case, the series of processes of the training device 3 is carried out by the computer systems of the plurality of computers.

### [Evaluation device 4]

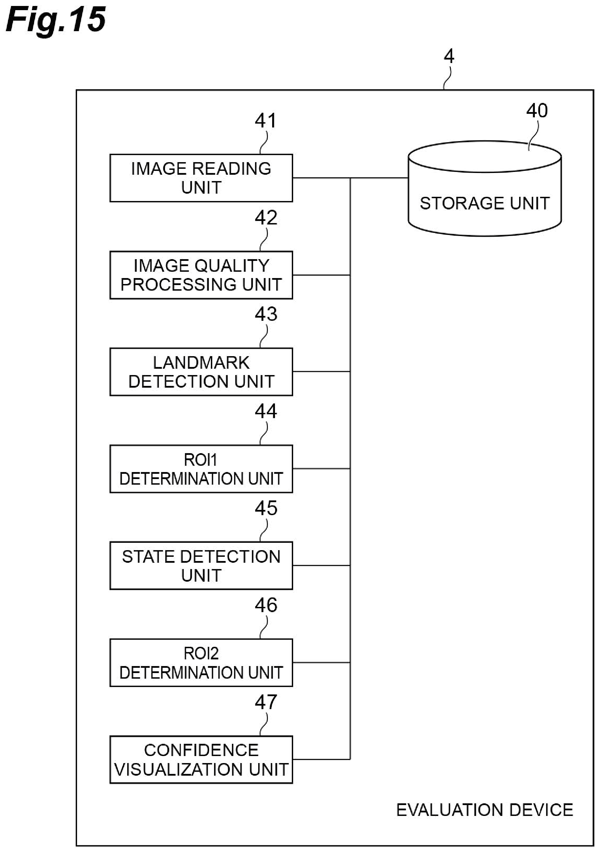

The evaluation device 4 is a computer device that performs various evaluations based on various detection models trained by the training device 3. The evaluation device 4 (or image processing system 1) may be a computer device that supports diagnosis of a joint state based on a joint image. Figure 15 shows a diagram illustrating an example of a functional configuration of the evaluation device 4. As shown in Figure 15, the evaluation device 4 comprises a storage unit 40, an image reading unit 41, an image quality processing unit 42, a landmark detection unit 43, a ROI1 determination unit 44, a state detection unit 45, a ROI2 determination unit 46, and a confidence visualization unit 47.

The functional blocks of the evaluation device 4 are assumed to function in the evaluation device 4, but the present invention is not limited thereto. For example, a part of the functional blocks of the evaluation device 4 may be a computer device different from the evaluation device 4, and function while appropriately sending and receiving information between itself and the evaluation device 4 in a computer device (for example, the preparation device 2, the training device 3 or the display device 5) that is network-connected to the evaluation device 4. A part of the functional blocks of the evaluation device 4 may be absent, a plurality of functional blocks may be integrated into one functional block, or one functional block may be divided into a plurality of functional blocks.

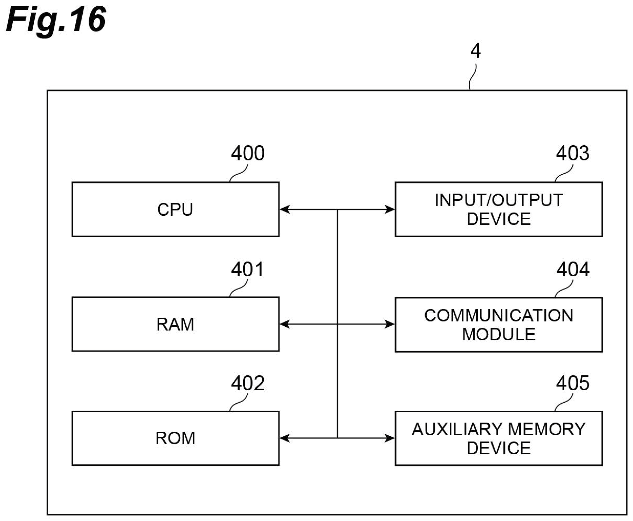

Figure 16 shows a diagram illustrating an example of a hardware configuration of a computer used in the evaluation device 4. Physically, the evaluation device 4 is configured as a computer system comprising a CPU 400, a RAM 401 and a ROM 402, an input/output device 403 such as a keyboard, a microphone or a display, a communication module 404 which is a data transmitting and receiving device, and an auxiliary memory device 405 such as a hard disk or a SSD, as shown in Figure 16. There may be a plurality of CPUs 400, RAMs 401, ROMs 402, input/output devices 403, communication modules 404 and auxiliary memory devices 405. The functions of the functional blocks shown in Figure 15 are performed by causing predetermined computer software to be read on hardware such as the CPU 400 and the RAM 401 shown in Figure 16, thereby operating the input/output device 403 and the communication module 404 under control of the CPU 400, and reading and writing data in the RAM 401 and the auxiliary memory device 405.

Hereinafter, the functions of the evaluation device 4 shown in Figure 15 will be described.

The storage unit 40 stores arbitrary information used or output in, for example, a process in the evaluation device 4. The storage unit 40 may store information calculated by the functions of the evaluation device 4. The information stored by the storage unit 40 may be appropriately referred to by the functions of the evaluation device 4, or may be appropriately referred to via a network by the functions of the preparation device 2, the training device 3 or the display device 5.

The image reading unit 41 reads an evaluable joint image which is a joint image to be evaluated by the evaluation device 4. The image reading unit 41 may read an evaluable joint image using the input/output device 403, or may read an evaluable joint image via a network from another device or the like using the communication module 404. For example, the image reading unit 41 may read an evaluable joint image from a joint image formation device (device that forms a joint image) which is another device. The image reading unit 41 may allow the read evaluable joint image to be stored by the storage unit 40, or output the read evaluable joint image to another functional block of the evaluation device 4.

The image quality processing unit 42 may adjust the image quality of an evaluable joint image by, for example, performing image quality enhancement (gradation adjustment, noise removal or the like) on the evaluable joint image. The image quality processing unit 42 may adjust the image quality of an evaluable joint image read by the image reading unit 41. More specifically, the image quality processing unit 42 may adjust the image quality of an evaluable joint image read by the image reading unit 41 and stored by the storage unit 40, or may adjust the image quality of an evaluable joint image read by the image reading unit 41 and output to the image quality processing unit 42 from the image reading unit 41. The image quality processing unit 42 may allow the evaluable joint image subjected to adjustment of image quality to be stored by the storage unit 40, or output the evaluable joint image to another functional block of the evaluation device 4.

The landmark detection unit 43 detects a joint landmark in an evaluable joint image. The landmark detection unit 43 may detect a joint landmark in an evaluable joint image read by the image reading unit 41, or may detect a joint landmark in an evaluable joint image subjected to adjustment of image quality by the image quality processing unit 42. More specifically, the landmark detection unit 43 may detect a joint landmark in an evaluable joint image read by the image reading unit 41 and stored by the storage unit 40, may detect a joint landmark in an evaluable joint image read by the image reading unit 41 and output to the landmark detection unit 43 from the image reading unit 41, may detect a joint landmark in an evaluable joint image subjected to adjustment of image quality by the image quality processing unit 42 and stored by the storage unit 40, or may detect a joint landmark in an evaluable joint image subjected to adjustment of image quality by the image quality processing unit 42 and output to the landmark detection unit 43 from the image quality processing unit 42.

The landmark detection unit 43 may detect two or more joint landmarks in an evaluable joint image. One of the two or more joint landmarks detected may be a bone, with another being a different bone, cartilage, fat tissue, a joint capsule or muscle. The landmark detection unit 43 may perform detection using a landmark detection model which, upon input of the joint image, detects two or more joint landmarks in the joint image. The landmark detection unit 43 may also detect types of the joint landmarks.

In detection of a joint landmark by the landmark detection unit 43, it may be impossible to detect the joint landmark or the confidence may be low. In this case, based on the relative position of a learned joint landmark, the positions of other landmarks may be estimated or amended.

The landmark detection unit 43 may add landmark detection information, which is information on the detected joint landmark, to an evaluable joint image, and then allow the evaluable joint image to be stored by the storage unit 40, or output the evaluable joint image to another functional block of the evaluation device 4.

The ROI1 determination unit 44 determines, in an evaluable joint image, ROI1 which is a region of interest for the joint, based on a joint landmark detected by the landmark detection unit 43. More specifically, ROI1 is determined in an evaluable joint image based on a joint landmark shown by landmark detection information output to the ROI1 determination unit 44 from the landmark detection unit 43. The ROI1 determination unit 44 may determine, in an evaluable joint image, ROI1 based on two or more joint landmarks detected by the landmark detection unit 43. The ROI1 determination unit 44 may determine ROI1 based further on the types of the joint landmarks detected by the landmark detection unit 43. The ROI1 determination unit 44 may determine ROI1 based on a distance from the center of a joint landmark detected by the landmark detection unit 43 (each of two or more joint landmarks detected by the landmark detection unit 43) to the boundary line of ROI1. The method for determining ROI1 by the ROI1 determination unit 44 is similar to the method for determining ROI1 by the ROI1 determination unit 34.

The ROI1 determination unit 44 may allow ROI1 information, which is information on determined ROI1, to be stored by the storage unit 40, or output the ROI1 information to another functional block of the evaluation device 4.

The state detection unit 45 detects a joint state based on a local joint image which is an image of the inside of ROI1 determined by the ROI1 determination unit 44 in an evaluable joint image. The evaluable joint image is similar to the evaluable joint image used by the landmark detection unit 43. The local joint image may be an image of the inside of ROI1 shown by ROI1 information stored by the storage unit 40, or an image of the inside of ROI1 shown by ROI1 information output to the state detection unit 45 from the ROI1 determination unit 44, in an evaluable joint image. The state detection unit 45 may detect a joint state using a state detection model which, upon input of a local joint image, detects the joint state of a joint shown by the local joint image.

The state detection unit 45 may add joint state detection information, which is information on the detected joint state, to a local joint image or an evaluable joint image, and then allow the joint image to be stored by the storage unit 40, or output the joint image to another functional block of the evaluation device 4.

When the joint state is abnormal, bleeding, synovitis or arthrosis, the joint state detection unit 45 may further indicate a relevant area in a local joint image.

The joint may be a foot joint, the joint landmark may be a tibia, a fibula, a talus or fat tissue, and the joint state may indicate normality, abnormality, bleeding, synovitis or arthrosis. The joint may be a knee joint, the joint landmark may be a femur, a tibia, a fibula, a patella or fat tissue, and the joint state may indicate normality, abnormality, bleeding, synovitis or arthrosis. The joint may be an elbow joint, the joint landmark may be a humerus, a radius, an ulna or fat tissue, and the joint state may indicate normality, abnormality, bleeding, synovitis or arthrosis.

In case where the state detection unit 45 detects a joint state indicating abnormality, the ROI2 determination unit 46 determines (cuts out, detects or identifies) ROI2 which is a region of interest which includes an abnormal area in a local joint image. For example, when joint state detection information output from the state detection unit 45 to the ROI2 determination unit 46 indicates that a joint state indicating abnormality has been detected, the ROI2 determination unit 46 determines a rectangular region including a relevant area in a local joint image, which is indicated by the joint state, as ROI2.

The ROI2 determination unit 46 may allow ROI2 information, which is information on determined ROI2, to be stored by the storage unit 40, or output the ROI2 information to another functional block of the evaluation device 4.

The confidence visualization unit 47 visualizes a portion of a local joint image, which is influenced in detection of the joint state by the state detection model, in a display mode corresponding to a degree of the influence (evidence for evaluation, confidence or evaluation confidence). For the visualization by the confidence visualization unit 47, Grad-CAM algorithm that is a conventional technique may be used. The confidence visualization unit 47 may perform display in a display mode where display is performed by a heat map, or normal areas are displayed in blue and abnormal areas are displayed in red. With the confidence visualization unit 47 instructing a later-described result display unit 51 of the display device 5 to provide visualization, the practitioner of visualization may be the result display unit 51.

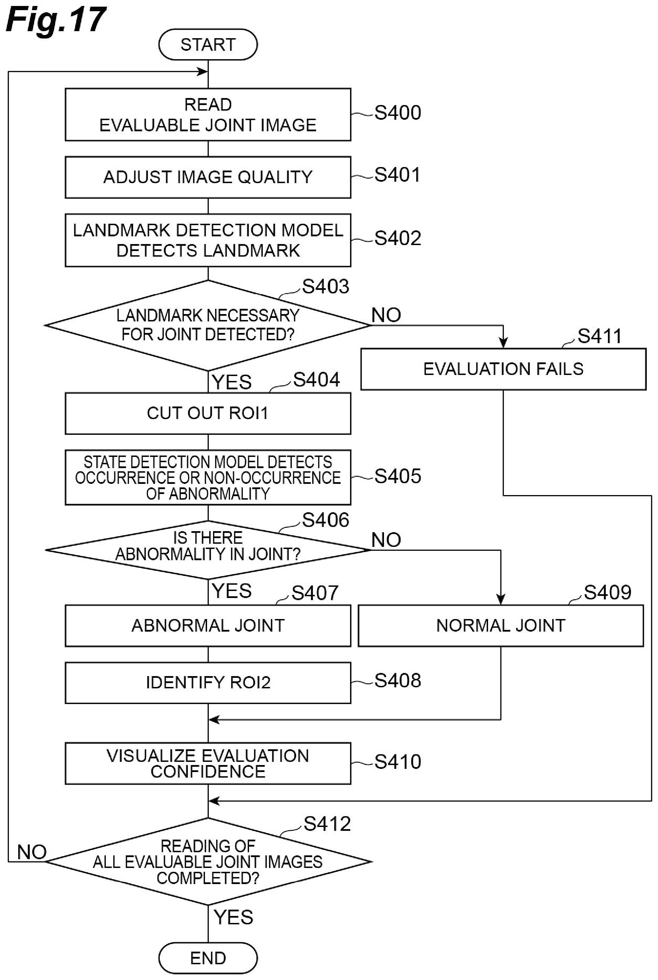

Figure 17 shows a flowchart illustrating an example of a process executed by the evaluation device 4 (image processing method). More specifically, Figure 17 shows a flowchart illustrating a process for determining whether or not the joint has an abnormality in an evaluable joint image.

First, the image reading unit 41 reads an evaluable joint image (step S400). Next, the image quality processing unit 42 adjusts the image quality of the evaluable joint image read in step S400 (step S401). Step S401 may be omitted. Next, the landmark detection unit 43 detects a joint landmark in the evaluable joint image subjected to adjustment of image quality in step S401 (the evaluable joint image read in step S400 when step S401 is omitted; the same applies hereinafter) (step S402). Next, the evaluation device 4 determines whether or not a landmark necessary for the joint has been detected by detection in step S402 (step S403). For example, whether or not ROI1 can be determined by at least one of the type and the number of the landmarks detected in step S402 is determined. For example, ROI1 can be determined when the boundary line of ROI1 (see Figure 10) can be estimated by two or more landmarks, and ROI1 cannot be determined when the boundary line cannot be estimated.

When it is determined in step S403 that the landmark has been detected (S403: YES), the ROI1 determination unit 44 determines, based on the joint landmark detected in step S402, ROI1 in the evaluable joint image subjected to adjustment of image quality in step S401 (step S404). Next, the state detection unit 45 detects a joint state (whether or not there is an abnormality) based on a local joint image which is an image of the inside of ROI1 determined in step S404 in the evaluable joint image subjected to adjustment of image quality in step S401 (step S405), followed by determination of whether or not the joint has an abnormality (step S406).

When it is determined in step S406 that the joint has an abnormality (S406: YES), it can be seen that the local joint image includes an abnormal joint (step S407). Next, the ROI2 determination unit 46 determines ROI2 which is a region of interest which includes an abnormal area in the local joint image (step S408). When it is determined in step S406 that the joint does not have an abnormality (S406: NO), it can be seen that the local joint image includes a normal joint (the local joint image does not include an abnormal joint) (step S409). Subsequent to step S408 or step S409, the confidence visualization unit 47 visualizes an evaluation confidence (step S410).

When it is determined in step S403 that the landmark has not been detected (S403: NO), the evaluation fails (step S411). Subsequent to step S410 or step S411, the evaluation device 4 determines whether or not reading of all evaluable joint images has been completed (step S412). When it is determined in step S412 that the reading has been completed (step S412: YES), the process is terminated. When it is determined in step S412 that the reading has not been completed (S412: NO), the processes of step S400 and the subsequent steps are repeated for the remaining evaluable joint images.

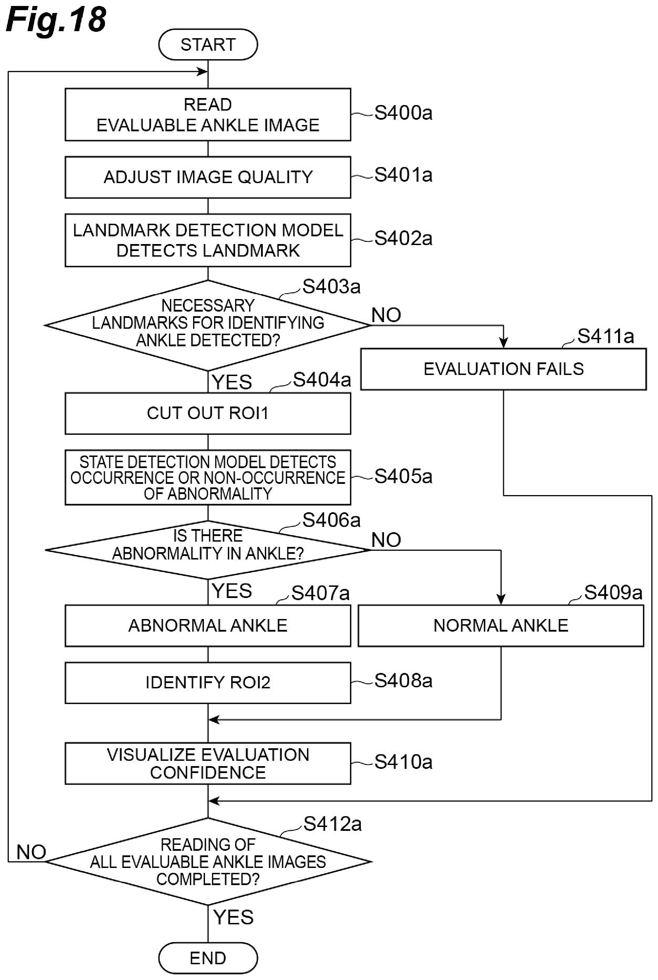

Figure 18 shows a flowchart illustrating another example of a process executed by the evaluation device 4. More specifically, Figure 18 shows, with respect to the flowchart shown in Figure 17, a specific example in which bleeding in an ankle is evaluated. For example, in Figure 18, the evaluable joint image in Figure 17 is replaced by a evaluable ankle image focused on an ankle. When the joint is a foot joint, a tibia, a talus and fat tissue may be used as landmarks.

First, the image reading unit 41 reads an evaluable ankle image (step S400a). Next, the image quality processing unit 42 adjusts the image quality of the evaluable ankle image read in step S400a (step S401a). Step S401a may be omitted. Next, the landmark detection unit 43 detects a joint landmark in the evaluable ankle image subjected to adjustment of image quality in step S401a (the evaluable ankle image read in step S400a when step S401a is omitted; the same applies hereinafter) (step S402a). Next, the evaluation device 4 determines whether or not a necessary landmark for identifying the ankle has been detected by detection in step S402a (step S403a).

When it is determined in step S403a that the landmark has been detected (S403a: YES), the ROI1 determination unit 44 determines, based on the joint landmark detected in step S402a, ROI1 in the evaluable ankle image subjected to adjustment of image quality in step S401a (step S404a). Next, the state detection unit 45 detects a joint state (whether or not there is an abnormality) based on a local joint image which is an image of the inside of ROI1 determined in step S404a in the evaluable ankle image subjected to adjustment of image quality in step S401a (step S405a), followed by determination of whether or not the ankle has an abnormality (step S406a).

When it is determined in step S406a that the joint has an abnormality (S406a: YES), it can be seen that the local joint image includes an abnormal ankle (step S407a). Next, the ROI2 determination unit 46 determines ROI2 which is a region of interest which includes an abnormal area in the local joint image (step S408a). When it is determined in step S406a that the joint does not have an abnormality (S406a: NO), it can be seen that the local joint image includes a normal ankle (the local joint image does not include an abnormal ankle) (step S409a). Subsequent to step S408a or step S409a, the confidence visualization unit 47 visualizes an evaluation confidence (step S410a).

When it is determined in step S403a that the landmark has not been detected (S403a: NO), the evaluation fails (step S411a). Subsequent to step S410a or step S411a, the evaluation device 4 determines whether or not reading of all evaluable ankle images has been completed (step S412a). When it is determined in step S412a that the reading has been completed (step S412a: YES), the process is terminated. When it is determined in step S412a that the reading has not been completed (S412a: NO), the processes of step S400a and the subsequent steps are repeated for the remaining evaluable ankle images.

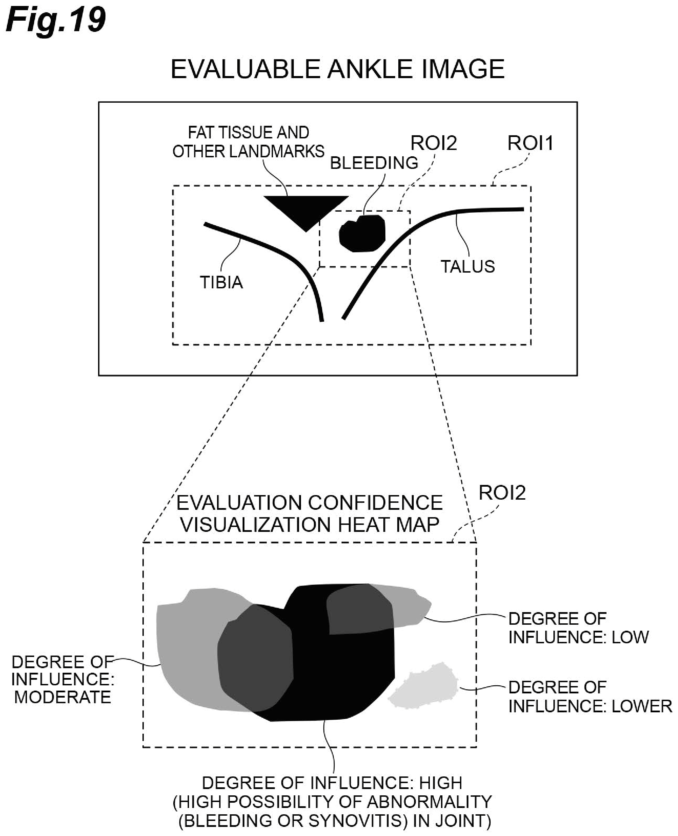

Figure 19 illustrates an example of a training ankle image which is prepared by the process shown in Figure 18. In the training ankle image shown in Figure 19, ROI1 cut out in step S404a, ROI2 cut out in step S408a and the evaluation confidence visualized in step S410a (evaluation confidence visualization heat map) of Figure 18 are shown. For the evaluation confidence, the degree to which the evaluation result is influenced by the feature of a region with respect to an abnormality (bleeding) is shown as high (high possibility of abnormality (bleeding or synovitis) in the joint), "moderate", "low" or "lower", depending on a gray level of the region.

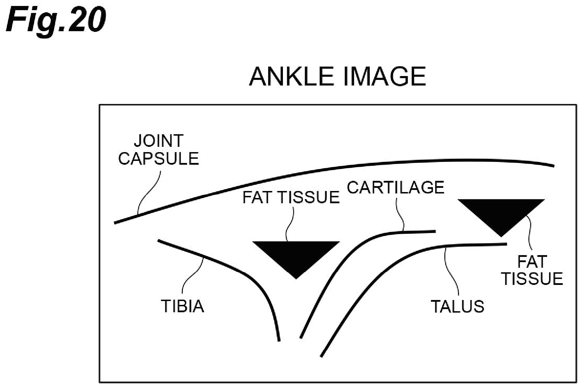

Examples of joint landmarks of the joint image will be described with reference to Figures 20 to 24.

Figure 20 shows a diagram illustrating an example of a joint landmark of an ankle image. Figure 20 shows a joint capsule, a tibia, fat tissue, cartilage and a talus as joint landmarks of the ankle image.

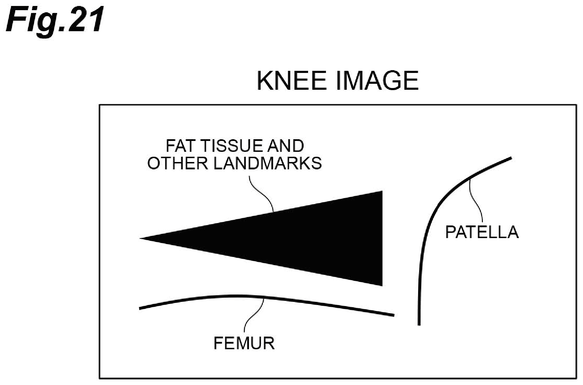

Figure 21 shows a diagram illustrating an example of a joint landmark of a knee image. Figure 21 shows a femur, a patella and fat tissue as joint landmarks of the knee image.

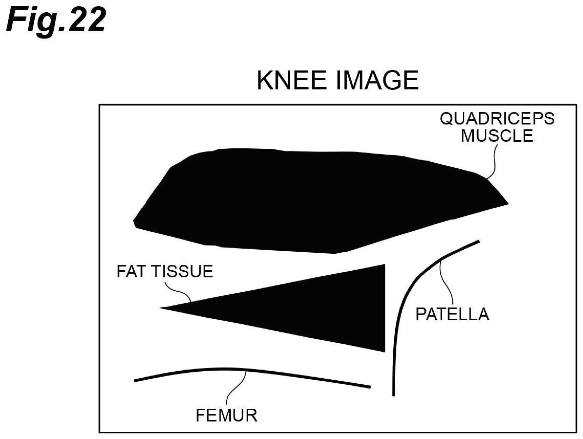

Figure 22 shows a diagram illustrating another example of a joint landmark of a knee image. Figure 22 shows quadriceps muscle, fat tissue, a femur and a patella as joint landmarks of the knee image.

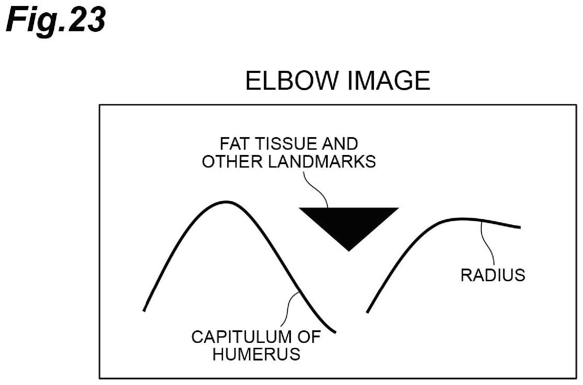

Figure 23 shows a diagram illustrating an example of a joint landmark of an elbow image. Figure 23 shows a capitulum of humerus, a radius and fat tissue as joint landmarks of the elbow image.

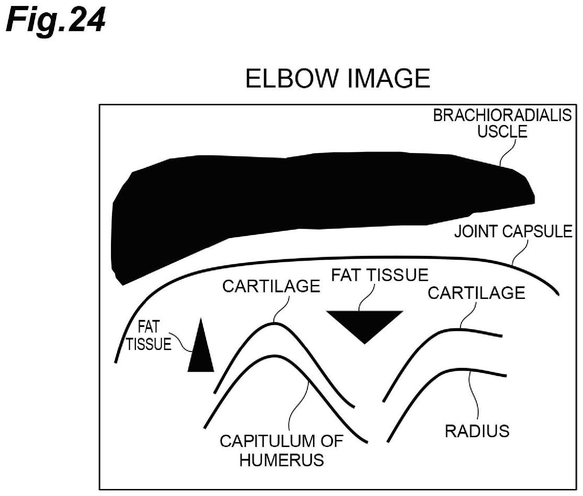

Figure 24 shows a diagram illustrating another example of a joint landmark of an elbow image. Figure 24 shows brachioradialis muscle, a joint capsule, fat tissue, cartilage, a capitulum of humerus and a radius as joint landmarks of the elbow image.

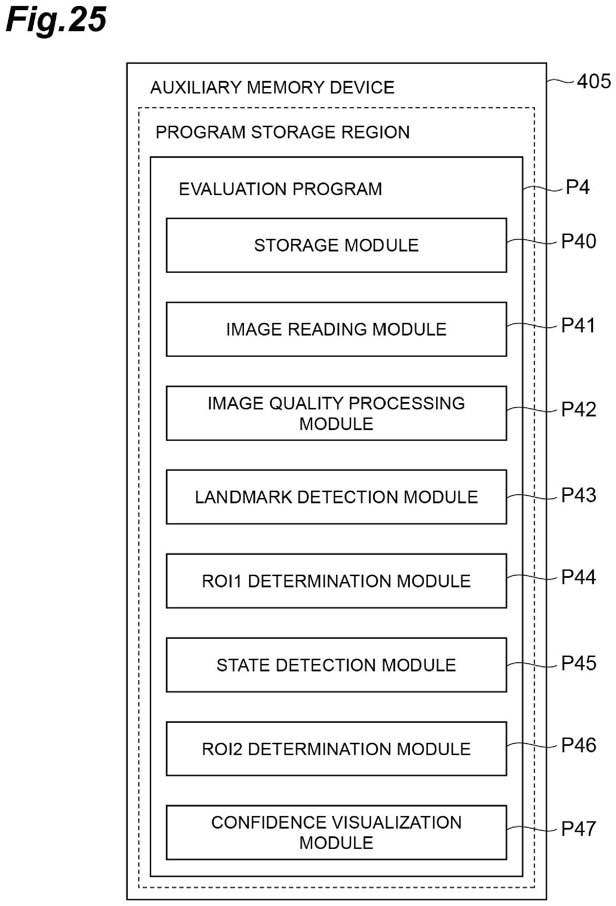

Next, an evaluation program P4 (image processing program) for causing a computer to execute a series of processes by the evaluation device 4 will be described. For example, as shown in Figure 25, the evaluation program P4 is stored in a program storage region formed in the auxiliary memory device 405 of the evaluation device 4.

The evaluation program P4 comprises a storage module P40, an image reading module P41, an image quality processing module P42, a landmark detection model P43, a ROI1 determination module P44, a state detection module P45, a ROI2 determination module P46, and a confidence visualization module P47. Functions that are performed by executing the storage module P40, the image reading module P41, the image quality processing module P42, the landmark detection module P43, the ROI1 determination module P44, the state detection module P45, the ROI2 determination module P46 and the confidence visualization module P47 are similar, respectively, to the functions of the storage unit 40, the image reading unit 41, the image quality processing unit 42, the landmark detection unit 43, the ROI1 determination unit 44, the state detection unit 45, the ROI2 determination unit 46 and the confidence visualization unit 47 of the evaluation device 4. The evaluation program P4 is a program for causing the evaluation device 4 (one or more CPUs thereof) to function as the storage unit 40, the image reading unit 41, the image quality processing unit 42, the landmark detection unit 43, the ROI1 determination unit 44, the state detection unit 45, the ROI2 determination unit 46 and the confidence visualization unit 47.

A part or the whole of the evaluation device 4 may be transmitted via a transmission medium such as a communication line, and received by another equipment, followed by being stored (as well as being installed). The modules of the evaluation device 4 may be installed not in one computer, but in any of a plurality of computers. In this case, the series of processes of the evaluation device 4 is carried out by the computer systems of the plurality of computers.

Next, the action and effect of the evaluation device 4 will be described.

The evaluation device 4 (image processing system 1) comprises the landmark detection unit 43 that detects, in a joint image which is an in vivo image of a human joint, two or more joint landmarks, each of which is a landmark for the joint, and the ROI1 determination unit 44 that determines, in the joint image, ROI1 for the joint based on the two or more joint landmarks detected. In this configuration, ROI1 is determined based on the two or more joint landmarks detected. In this way, more accurate ROI1 can be determined.

The landmark detection unit 43 may perform detection using a landmark detection model which, upon input of the joint image, detects the two or more joint landmarks in the joint image. In this configuration, two or more joint landmarks can be more reliably and accurately detected by using a landmark detection model.

The landmark detection model may be a model trained based on training data comprising a pair of the joint image and an image in which the two or more joint landmarks in the joint image are annotated. In this configuration, the landmark detection model trained based on the training data is capable of more reliably and accurately detecting two or more joint landmarks.

The landmark detection unit 43 may detect landmarks as well as types of the joint landmarks, and the ROI1 determination unit 44 may determine ROI1 based further on the detected types of the joint landmarks. In this configuration, ROI1 is determined based further on types of the joint landmarks. In this way, more accurate ROI1 can be determined.

One of the two or more joint landmarks detected may be a bone, with another being a different bone, cartilage, fat tissue, a joint capsule or muscle. In this configuration, more accurate ROI1 can be determined based on a more specific joint landmark.

The joint may be a joint of a coagulation defect patient or a rheumatoid arthritis patient, and is preferably a joint of hemophilia patient. In this configuration, ROI1 in a joint image for a coagulation defect patient or a rheumatoid arthritis patient (preferably a hemophilia patient) can be determined.

The joint image may be an ultrasound image or an X-ray image. In general, echography or X-ray radiography can be conveniently conducted on an outpatient basis, and therefore, in this configuration, ROI1 can be conveniently determined on an outpatient basis, for example.

The joint image may be an image having enhanced image quality. In this configuration, two or more joint landmarks can be more accurately detected based on a joint image having enhanced image quality. In this way, more accurate ROI1 can be determined.

The joint landmark may be a tibia, a talus, a fibula, a femur, a patella, a humerus, a radius, an ulna, cartilage, fat tissue, a joint capsule, or muscle. In this configuration, more accurate ROI1 can be determined based on a more specific joint landmark.

The ROI1 determination unit 44 may determine ROI1 based on the distance from the center of the joint landmark to the boundary line of ROI1. In this aspect, ROI1 can be more reliably determined.

The evaluation device 4 may further comprise the state detection unit 45 that detects a joint state, which is a state of the joint, based on a local joint image which is an image of the inside of the determined ROI1 in the joint image. In this configuration, a more accurate joint state can be detected based on a local joint image. In this way, a user can know a more accurate joint state.

The state detection unit 45 may perform detection using a state detection model which, upon input of the local joint image, detects the joint state of the joint shown by the local joint image. In this configuration, a joint state can be more reliably and accurately detected by using a state detection model.

The state detection model may be a model trained based on training data comprising a pair of the local joint image and the local joint image with which a normality label is associated or the local joint image in which an abnormality label is associated with an abnormal area of the joint shown by the local joint image. In this configuration, the state detection model trained based on the training data is capable of more reliably and accurately detecting a joint state.

The evaluation device 4 may further comprise the confidence visualization unit 47 that visualizes a portion of the local joint image, which is influenced in detection of the joint state by the state detection model, in a display mode corresponding to a degree of the influence. In this configuration, a user can know a joint state in more detail in the display mode.

The evaluation device 4 may indicate that the joint state is normal, abnormal, bleeding, synovitis or arthrosis. In this aspect, a more specific joint state can be output. In this way, a user can know a more specific joint state.

When indicating that the joint state is abnormal, bleeding, synovitis or arthrosis, the evaluation device 4 may further indicate a relevant area in the local joint image. In this aspect, abnormality, bleeding, synovitis or arthrosis, as well as a relevant area in the local joint image can be output. In this way, a user can know a more specific joint state.

The joint may be a foot joint, the joint landmark may be a tibia, a fibula, a talus or fat tissue, and the joint state may indicate normality, abnormality, bleeding, synovitis or arthrosis. In this aspect, more accurate ROI1 can be determined based on two or more specific joint landmarks in a foot joint image. A specific joint state can be output based on the determined ROIC. In this way, a user can know a more accurate and specific joint state of a foot joint.

The joint may be a knee joint, the joint landmark may be a femur, a tibia, a fibula, a patella or fat tissue, and the joint state may indicate normality, abnormality, bleeding, synovitis or arthrosis. In this aspect, more accurate ROI1 can be determined based on two or more specific joint landmarks in a knee joint image. A specific joint state can be output based on the determined ROI1. In this way, a user can know a more accurate and specific joint state of a knee joint.

The joint may be an elbow joint, the joint landmark may be a humerus, a radius, an ulna or fat tissue, and the joint state may indicate normality, abnormality, bleeding, synovitis or arthrosis. In this aspect, more accurate ROI1 can be determined based on two or more specific joint landmarks in an elbow joint image. A specific joint state can be output based on the determined ROI1. In this way, a user can know a more accurate and specific joint state of an elbow joint.

The image processing system 1 or the evaluation device 4 may support diagnosis of the joint state based on the joint image. In this aspect, diagnosis of a joint state can be supported based on a joint image.

### [Display device 5]

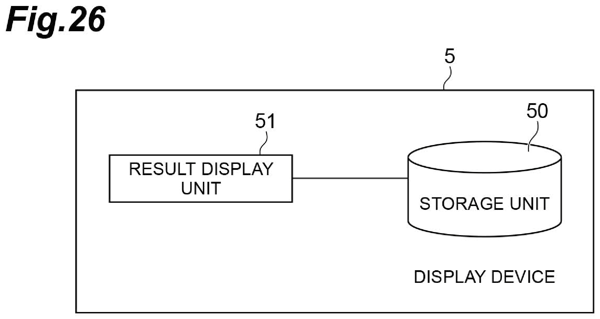

The display device 5 is a computer device that displays results of evaluation by the evaluation device 4. Figure 26 shows a diagram illustrating an example of a functional configuration of the display device 5. As shown in Figure 26, the display device 5 comprises a storage unit 50, and a result display unit 51.

The functional blocks of the display device 5 are assumed to function in the display device 5, but the present invention is not limited thereto. For example, a part of the functional blocks of the display device 5 may be a computer device different from the display device 5, and function while appropriately sending and receiving information between itself and the display device 5 in a computer device (for example, the preparation device 2, the training device 3 or the evaluation device 4) that is network-connected to the display device 5. A part of the functional blocks of the display device 5 may be absent, a plurality of functional blocks may be integrated into one functional block, or one functional block may be divided into a plurality of functional blocks.

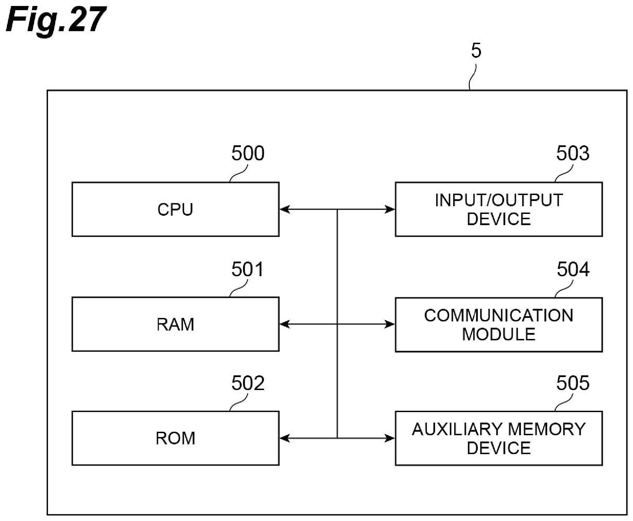

Figure 27 shows a diagram illustrating an example of a hardware configuration of a computer used in the display device 5. Physically, the display device 5 is configured as a computer system comprising a CPU 500, a RAM 501 and a ROM 502, an input/output device 503 such as a keyboard, a microphone or a display, a communication module 504 which is a data transmitting and receiving device, and an auxiliary memory device 505 such as a hard disk or a SSD, as shown in Figure 27. There may be a plurality of CPUs 500, RAMs 501, ROMs 502, input/output devices 503, communication modules 504 and auxiliary memory devices 505. The functions of the functional blocks shown in Figure 26 are performed by causing predetermined computer software to be read on hardware such as the CPU 500 and the RAM 501 shown in Figure 27, thereby operating the input/output device 503 and the communication module 504 under control of the CPU 500, and reading and writing data in the RAM 501 and the auxiliary memory device 505.

Hereinafter, the functions of the display device 5 shown in Figure 26 will be described.

The storage unit 50 stores arbitrary information used or output in, for example, a process in the display device 5. The storage unit 50 may store information calculated by the functions of the display device 5. The information stored by the storage unit 50 may be appropriately referred to by the functions of the display device 5, or may be appropriately referred to via a network by the functions of the preparation device 2, the training device 3 or the training device 4.

The result display unit 51 responds to an instruction from the confidence visualization unit 47 of the evaluation device 4 to visualize and display a portion of a local joint image, which is influenced in detection of the joint state by the state detection model, in a display mode corresponding to a degree of the influence. The result display unit 51 may display a joint landmark on an evaluable joint image detected by the landmark detection unit 43 of the evaluation device 4, ROI1 on an evaluable joint image, which is determined by the ROI1 determination unit 44 of the evaluation device 4, information about the joint state (for example, a result of the joint evaluated as being abnormal) on an evaluable joint image, which is detected by the state detection unit 45 of the evaluation device 4, or ROI2 on a local joint image, which is determined by the ROI2 determination unit 46 of the evaluation device 4. The result display unit 51 may display the output results of the functions of the preparation device 2, the training device 3 and the evaluation device 4 at an arbitrary time.

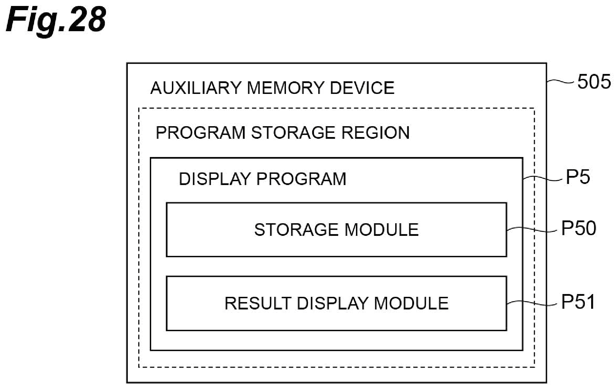

Next, a display program P5 for causing a computer to execute a series of processes by the display device 5 will be described. For example, as shown in Figure 28, the display program P5 is stored in a program storage region formed in the auxiliary memory device 505 of the display device 5.

The display program P5 comprises a storage module P50 and a result display module P51. Functions that are performed by executing the storage module P50 and the result display module P51 are similar, respectively, to the functions of the storage unit 50 and the result display unit 51 of the display device 5. The display program P5 is a program for causing the display device 5 (one or more CPUs thereof) to function as the storage unit 50 and the result display unit 51.

A part or the whole of the display device 5 may be transmitted via a transmission medium such as a communication line, and received by another equipment, followed by being stored (as well as being installed). The modules of the display device 5 may be installed not in one computer, but in any of a plurality of computers. In this case, the series of processes of the display device 5 is carried out by the computer systems of the plurality of computers.

The background will be described. Hemophilia is a congenital bleeding disease, a major problem of which is intra-articular bleeding. The occurrence of intra-articular bleeding is particularly common in the knee joint, the elbow joint and the foot joint. Repeated intra-articular bleeding leads to hemophilic arthrosis, resulting in difficulty with ambulation. Selection of a hemostatic therapy for hemophilia depends mainly on subjective evaluation by a patient (e.g., pain and discomfort of a joint). Evaluation of a joint state has not been widely available. Joint echography can be conveniently conducted on an outpatient basis, but has not been widely available due to difficulty of imaging diagnosis of hemostatic arthrosis.

The image processing system 1 can construct an AI algorithm for estimating occurrence or non-occurrence of joint bleeding and synovitis using a joint ultrasound image, and evaluate diagnostic accuracy thereof. The image processing system 1 provides support of diagnosis on a joint ultrasound image by AI to perform appropriate therapeutic intervention based on objective evaluation of intra-articular bleeding/non-bleeding using a joint ultrasound image, instead of subjective evaluation by a patient, so that healthy and active lives of hemophilia patients can be achieved. Establishment of a convenient method for diagnosis by joint echography with the image processing system 1 can be expected to lead to increased participation by physicians who are not specialized in the relevant field.

The image processing system 1 enables synovitis in the ankle, knee and elbow joints of a patient with congenital hemophilia A to be identified by AI. The image processing system 1 has a system configuration in which the confidence visualization unit 47 is added, so that evidence and influence of AI model evaluation can be displayed, and more accurate information can be given to a physician. It is generally known that the interpretation ability of some AI model algorithms (in particular, Deep Learning) is not high, and it is often the case that although the classification result (e.g., abnormal or normal) is correct, feature values of wrong areas are used for classification. The image processing system 1 provides an advantage that a physician re-evaluates AI model evaluation results (landmarks, abnormalities and evidence for visualization thereof) from a clinical standpoint, and can make a more correct clinical judgment. The image processing system 1 is characterized in that landmarks and groups of landmarks of multiple types are annotated, and training is performed. The image processing system 1, which detects landmarks and groups of landmarks of multiple types, then cuts out an ROI (region of interest) for the joint, and inputs only the local image of the ROI (region of interest) to a next processing module, thus has the advantage of being higher in accuracy and taking a shorter processing time as compared to direct evaluation of abnormality over the entire image. The image processing system 1 annotates a bone surface in a training stage, which allows a physician to easily identify a normal joint and identify bleeding and synovitis.

### [Reference Signs List]

1···image processing system, 2···preparation device, 3···training device, 4···evaluation device, 5···display device, 20···storage unit, 21···image reading unit, 22···landmark input unit, 23···joint state input unit, 24···input amendment unit, 30···storage unit, 31···image reading unit, 32···image processing unit, 33···landmark detection model training unit, 34···ROI1 determination unit, 35···state detection model training unit, 40···storage unit, 41···image reading unit, 42···image treating unit, 43···landmark detection unit, 44···ROI1 determination unit, 45···state detection unit, 46···ROI2 determination unit, 47···confidence visualization unit, 200·300·400·500···CPU, 201·301·401·501···RAM, 202·302·402·502···ROM, 203·303·403·503···input/output device, 204·304·404·504···communication module, 205·305·405·505···auxiliary memory device, P2···preparation program, P3···training program, P4···evaluation program, P5···display program, P20···storage module, P21···image reading module, P22···landmark input module, P23···joint state input module, P24···input amendment module, P30···storage module, P31···image reading module, P32···image quality processing module, P33···landmark detection model training module, P34···ROI1 determination module, P35···state detection model training module, P40···storage module, P41···image reading module, P42···image quality processing module, P43···landmark detection module, P44···ROI1 determination module, P45···state detection module, P46···ROI2 determination module, P47···confidence visualization module, P50···storage module, P51···result display module

## Claims

1. An image processing system comprising:
a landmark detection unit that detects, in a joint image which is an in vivo image of a human joint, two or more joint landmarks, each of which is a landmark for the joint; and
a determination unit that determines, in the joint image, a region of interest for the joint based on the two or more joint landmarks detected.

2. The image processing system according to claim 1, wherein the landmark detection unit performs detection using a landmark detection model which, upon input of the joint image, detects the two or more joint landmarks in the joint image.

3. The image processing system according to claim 2, wherein the landmark detection model is a model trained based on training data comprising a pair of the joint image and an image in which the two or more joint landmarks in the joint image are annotated.

4. The image processing system according to any one of claims 1 to 3, wherein the landmark detection unit also detects types of the joint landmarks, and
the detection unit performs determination based further on the detected types of the joint landmarks.

5. The image processing system according to any one of claims 1 to 4, wherein one of the two or more joint landmarks detected is a bone, and another is a different bone, cartilage, fat tissue, a joint capsule or muscle.

6. The image processing system according to any one of claims 1 to 5, wherein the joint is a joint of a hemophilia patient.

7. The image processing system according to any one of claims 1 to 6, wherein the joint image is an ultrasound image or an X-ray image.

8. The image processing system according to any one of claims 1 to 7, wherein the joint image is an image having enhanced image quality.

9. The image processing system according to any one of claims 1 to 8, wherein the joint landmark is a tibia, a talus, a fibula, a femur, a patella, a humerus, a radius, an ulna, cartilage, fat tissue, a joint capsule, or muscle.

10. The image processing system according to any one of claims 1 to 9, wherein the determination unit determines the region of interest based on a distance from a center of the joint landmark to a boundary line of the region of interest.

11. The image processing system according to any one of claims 1 to 10, further comprising a state detection unit that detects a joint state which is a state of the joint, based on a local joint image which is an image of an inside of the determined region of interest in the joint image.

12. The image processing system according to claim 11, wherein the state detection unit performs detection using a state detection model which, upon input of the local joint image, detects the joint state of the joint shown by the local joint image.

13. The image processing system according to claim 12, wherein the state detection model is a model trained based on training data comprising a pair of the local joint image and the local joint image with which a normality label is associated or the local joint image in which an abnormality label is associated with an abnormal area of the joint shown by the local joint image.

14. The image processing system according to claim 12 or 13, further comprising a visualization unit that visualizes a portion of the local joint image, which is influenced in detection of the joint state by the state detection model, in a display mode corresponding to a degree of the influence.

15. The image processing system according to any one of claims 11 to 14, wherein the joint state indicates normality, abnormality, bleeding, synovitis or arthrosis.

16. The image processing system according to claim 15, wherein when indicating abnormality, bleeding, synovitis or arthrosis, the joint state further indicates a relevant area in the local joint image.

17. The image processing system according to any one of claims 11 to 16,
wherein the joint is a foot joint,
the joint landmark is a tibia, a fibula, a talus or fat tissue, and
the joint state indicates normality, abnormality, bleeding, synovitis or arthrosis.

18. The image processing system according to any one of claims 11 to 16,
wherein the joint is a knee joint,
the joint landmark is a femur, a tibia, a fibula, a patella or fat tissue, and
the joint state indicates normality, abnormality, bleeding, synovitis or arthrosis.

19. The image processing system according to any one of claims 11 to 16,
wherein the joint is an elbow joint,
the joint landmark is a humerus, a radius, an ulna or fat tissue, and
the joint state indicates normality, abnormality, bleeding, synovitis or arthrosis.

20. The image processing system according to any one of claims 11 to 19, wherein diagnosis of the joint state is supported based on the joint image.

21. An image processing program for causing a computer to function as
a landmark detection unit that detects, in a joint image which is an in vivo image of a human joint, two or more joint landmarks, each of which is a landmark for the joint, and
a determination unit that determines, in the joint image, a region of interest for the joint based on the two or more joint landmarks detected.

22. The image processing program according to claim 21, wherein the landmark detection unit performs detection using a landmark detection model which, upon input of the joint image, detects the two or more joint landmarks in the joint image.

23. The image processing program according to claim 22, wherein the landmark detection model is a model trained based on training data comprising a pair of the joint image and an image in which the two or more joint landmarks in the joint image are annotated.

24. The image processing program according to any one of claims 21 to 23, wherein the landmark detection unit also detects types of the joint landmarks, and
the detection unit performs determination based further on the detected types of the joint landmarks.

25. The image processing program according to any one of 21 to 24, wherein one of the two or more joint landmarks detected is a bone, and another is a different bone, cartilage, fat tissue, a joint capsule or muscle.

26. The image processing program according to any one of claims 21 to 25, wherein the joint is a joint of a hemophilia patient.

27. The image processing program according to any one of claims 21 to 26, wherein the joint image is an ultrasound image or an X-ray image.

28. The image processing program according to any one of claims 21 to 27, wherein the joint image is an image having enhanced image quality.

29. The image processing program according to any one of claims 21 to 28, wherein the joint landmark is a tibia, a talus, a fibula, a femur, a patella, a humerus, a radius, an ulna, cartilage, fat tissue, a joint capsule, or muscle.

30. The image processing program according to any one of claims 21 to 29, wherein the determination unit determines the region of interest based on a distance from a center of the joint landmark to a boundary line of the region of interest.

31. The image processing program according to any one of claims 21 to 30, for causing the computer to function further as a state detection unit that detects a joint state which is a state of the joint, based on a local joint image which is an image of an inside of the determined region of interest in the joint image.

32. The image processing program according to claim 31, wherein the state detection unit performs detection using a state detection model which, upon input of the local joint image, detects the joint state of the joint shown by the local joint image.

33. The image processing program according to claim 32, wherein the state detection model is a model trained based on training data comprising a pair of the local joint image and the local joint image with which a normality label is associated or the local joint image in which an abnormality label is associated with an abnormal area of the joint shown by the local joint image.

34. The image processing program according to claim 32 or 33, for causing the computer to function further as a visualization unit that visualizes a portion of the local joint image, which is influenced in detection of the joint state by the state detection model, in a display mode corresponding to a degree of the influence.

35. The image processing program according to any one of claims 31 to 34, wherein the joint state indicates normality, abnormality, bleeding, synovitis or arthrosis.

36. The image processing program according to claim 35, wherein when indicating abnormality, bleeding, synovitis or arthrosis, the joint state further indicates a relevant area in the local joint image.

37. The image processing program according to any one of claims 31 to 36,
wherein the joint is a foot joint,
the joint landmark is a tibia, a fibula, a talus or fat tissue, and
the joint state indicates normality, abnormality, bleeding, synovitis or arthrosis.

38. The image processing program according to any one of claims 31 to 36,
wherein the joint is a knee joint,
the joint landmark is a femur, a tibia, a fibula, a patella or fat tissue, and
the joint state indicates normality, abnormality, bleeding, synovitis or arthrosis.

39. The image processing program according to any one of claims 31 to 36,
wherein the joint is an elbow joint,
the joint landmark is a humerus, a radius, an ulna or fat tissue, and
the joint state indicates normality, abnormality, bleeding, synovitis or arthrosis.

40. The image processing program according to any one of claims 31 to 39, wherein diagnosis of the joint state is supported based on the joint image.

41. An image processing method executed by a computer, comprising:
a landmark detection step of detecting, in a joint image which is an in vivo image of a human joint, two or more joint landmarks, each of which is a landmark for the joint, and
a determination step of determining, in the joint image, a region of interest for the joint based on the two or more joint landmarks detected.

42. An image processing device comprising:
a landmark detection unit that detects, in a joint image which is an in vivo image of a human joint, two or more joint landmarks, each of which is a landmark for the joint; and
a determination unit that determines, in the joint image, a region of interest for the joint based on the two or more joint landmarks detected.

43. The image processing device according to claim 42, further comprising a state detection unit that detects a joint state which is a state of the joint, based on a local joint image which is an image of an inside of the determined region of interest in the joint image.
